# EUROPEAN PATENT SPECIFICATION

(11) **EP 3 068 364 B1**
(45) Date of publication and mention of the grant of the patent: **03.11.2021**
(21) Application number: 14863002.3
(22) Date of filing: 14.11.2014
(51) Int. Cl.: A61K 8/18, A61K 31/366, A61K 8/35, A61P 39/06, A61P 17/18, A61P 39/00

(54) **NAD+ PRECURSOR SELECTED FROM NMN, NAAD, NAMN, OR A PHARMACEUTICALLY ACCEPTABLE SALT THEREOF, FOR USE IN TREATING A SIDE EFFECT OF CHEMOTHERAPY AND/OR RADIOTHERAPY IN A SUBJECT**
NAD+ VORLÄUFER AUSGEWÄHLT VON NMN,NAAD,NAMN, ODER EINEM PHARMAZEUTISCHEN VERTRÄGLICHEN SALZ DAZU, ZUR BEHANDLUNG EINER NEBENWIRKUNG VON CHEMOTHERAPIE UND/ODER STRAHLENTHERAPIE IN EINEM PATIENTEN
PRÉCURSEUR DE NAD+ CHOISI PARMI LES NMN, NAAD,NAMN, OU SEL PHARMACEUTIQUEMENT ACCEPTABLE POUR LE TRAITEMENT D'UN EFFET SECONDAIRE DE LA RADIOTHERAPIE ET/OU LA CHIMIOTHERAPIE CHEZ UN SUJET

(30) Priority: 14.11.2013 AU 2013904415
(43) Date of publication of application: 21.09.2016
(73) Proprietor: Metro Biotech NSW Pty Ltd, Waterloo NSW 2017 (AU)
(72) Inventor: WU, Lindsay Edward, Waterloo, New South Wales 2017 (AU); SINCLAIR, David Andrew, Chestnut Hill, Massachusetts 02467 (US)
(74) Representative: Boult Wade Tennant LLP
(86) International application number: PCT/AU2014/001048
(87) International publication number: WO 2015/070280

(56) References cited:
- WO-A1-01/64211
- WO-A1-2009/108999
- WO-A1-2012/068539
- WO-A1-2013/090645
- WO-A1-2014/038873
- WO-A1-2016/176437
- WO-A2-2006/066244
- WO-A2-2008/011364
- WO-A2-2014/107730
- CN-A- 1 695 635
- CN-A- 102 631 385
- CN-A- 102 973 461
- US-A1- 2006 002 914
- US-A1- 2007 117 765
- US-B1- 7 012 086
- RITHIDECH K N ET AL: "Protective effect of apigenin on radiation-induced chromosomal damage in human lymphocytes", MUTATION RESEARCH. GENETIC TOXICOLOGY AND ENVIRONMENTAL MUTAGENESIS, ELSEVIER, AMSTERDAM, NL, vol. 585, no. 1-2, 1 August 2005 (2005-08-01), pages 96-104, XP027658886, ISSN: 1383-5718 [retrieved on 2005-08-01]
- CHUNG S. ET AL.: 'Regulation of SIRT 1 in cellular functions: role of polyphenols' ARCH. BIOCHEM. BIOPHYS. vol. 501, no. 1, 2010, pages 79 - 90, XP027262626
- DEVIPRIYA N. ET AL.: 'Quercetin ameliorates gamma radiation-induced DNA damage and biochemical changes in human peripheral blood lymphocytes' MUTATION RESEARCH vol. 654, 2008, pages 1 - 7, XP027262626
- BEGUM N. ET AL.: 'Apigenin ameliorates gamma radiation-induced cytogenetic alterations in cultured human blood lymphocytes' MUTATION RESEARCH vol. 747, 2012, pages 71 - 76, XP022825237
- YAN L. ET AL.: 'Quercetin inhibits left ventricular hypertrophy in spontaneously hypertensive rats and inhibits angiotensin II-induced H9C2 cells hypertrophy by enhancing PPAR-y expression and suppressing AP -1 activity' PLOS ONE vol. 8, no. 9, September 2013, page E72548, XP055344436
- LIN C. ET AL.: 'Combination of quercetin with radiotherapy enhances tumor radiosensitivity in vitro and in vivo' RADIOTHERAPY AND ONCOLOGY vol. 104, 2012, pages 395 - 400, XP055344438
- ESCANDE C. ET AL.: 'Flavonoid Apigenin Is an Inhibitor of the NAD+ ase CD 38. Implications for Cellular NAD+ Metabolism, Protein Acetylation, and Treatment of Metabolic Syndrome' DIABETES vol. 62, April 2013, pages 1084 - 1093, XP055344438
- YANG H. H. ET AL.: 'Quercetin-3-O-beta-D-glucuronide isolated from Polygonum aviculare inhibits cellular senescence in human primary cells' ARCH. PHARM. RES. vol. 37, 18 March 2014, pages 1219 - 1233, XP055166021
- JUN LI ET AL: "A conserved NAD + binding pocket that regulates protein-protein interactions during aging", SCIENCE, vol. 355, no. 6331, 24 March 2017 (2017-03-24), pages 1312-1317, XP055474350, US ISSN: 0036-8075, DOI: 10.1126/science.aad8242

## Description

### Field of the Invention

The invention relates to treating or preventing DNA damage and/or cellular senescence, and to treating and preventing conditions associated with DNA damage and/or cellular senescence.

### Background

Cellular senescence is the disruption of cell proliferation and function. During cellular senescence, there is a loss of the ability of the cell to proliferate, although the cell continues to remain viable and metabolically active.

Many cell types undergo cellular senescence following a large number of cycles of cell division. For example, when grown in culture, primary cells undergo cellular senescence after approximately 50 cell divisions. This barrier to further proliferation following many cycles of cell division has been termed replicative senescence. Replicative senescence is thought to be due to shortening of the cell's telomeres with each successive cell division, causing cells to reach a point (their so-called "Hayflick limit") at which a DNA damage response is triggered, leading ultimately to induction of proliferation arrest and cellular senescence.

Cellular senescence can also be induced in the absence of telomere loss or dysfunction. This type of cellular senescence, termed premature senescence, may result from, for example, DNA damage arising from chemotherapy, radiotherapy, or exposure to DNA damaging compounds or stimuli such as sunlight, UV light, and radiation. DNA damage may take the form of chromosomal dysfunction such as aneuploidy arising from unequal chromosome segregation during mitosis, DNA strand breaks, or chemical modification of DNA (e.g. alkylation). Cellular senescence may also be induced by a DNA damage response (DDR) which may or may not reflect actual DNA damage. Cellular senescence is characterized by, and may be induced by, changes in chromatin organization that induce changes in gene expression, such as for example, the "senescence-associated secretory phenotype ("SASP") in which senescent cells secrete inflammatory cytokines and mitokines that can damage or alter the surrounding tissue.

Studies have indicated that cellular senescence is associated with age-related conditions, including thinning of the epidermis, skin wrinkling, hair loss and greying hair, reduction in muscle thickness and muscle strength, increased incidence of inflammation, metabolic disturbances, loss of endurance, and age-associated diseases. In addition, cellular senescence is believed to contribute to damage to healthy tissues experienced during and following chemotherapy and/or radiotherapy, and the poor health effects post chemotherapy and/or radiotherapy.

Accordingly, preventing cells from undergoing cellular senescence, or preventing DNA damage, DNA damage response pathways or chromatin changes that would activate senescence, or reversing cellular senescence in cells which have undergone cellular senescence, would be advantageous to prevent or treat age and side effects of cancer treatments. Similarly, preventing cells from undergoing DNA damage and/or senescence in response to exposure to sunlight or exposure to DNA damaging chemicals may reduce skin aging, prevent skin cancer and improve cosmetic appearance.

### Summary

The invention is set out in the appended set of claims.

A first aspect provides treating or preventing DNA damage in a cell or cellular senescence of a cell or induction of the senescence associated secretory phenotype (SASP) in a cell, comprising contacting the cell with an NAD⁺ agonist.

A second aspect provides treating or preventing DNA damage or cellular senescence or induction of SASP in cells of a subject in need thereof, comprising administering to the subject an effective amount of an NAD⁺ agonist.

Alternatively, the second aspect provides use of an NAD⁺ agonist in the manufacture of a medicament for treating or preventing DNA damage or cellular senescence or induction of SASP in cells of a subject in need thereof, or an NAD⁺ agonist for use in treating or preventing DNA damage or cellular senescence or induction of SASP in cells of a subject in need thereof.

A third aspect provides reduction of the side effects of chemotherapy and/or radiotherapy in a subject in need thereof, comprising administering to the subject an effective amount of an NAD⁺ agonist.

Alternatively, a third aspect provides use of an NAD⁺ agonist in the manufacture of a medicament for reducing the side effects of chemotherapy and/or radiotherapy in a subject in need thereof, or an NAD⁺ agonist for use in reducing the side effects of chemotherapy and/or radiotherapy in a subject in need thereof.

A fourth aspect provide treating or preventing the effects of aging in a subject in need thereof, comprising administering to the subject an effective amount of an NAD⁺ agonist.

Alternatively, the fourth aspect provides use of an NAD⁺ agonist in the manufacture of a medicament for treating or preventing the effects of aging in a subject in need thereof, or an NAD⁺ agonist for use in treating or preventing the effects of aging in a subject in need thereof.

A fifth aspect provides reduction of the side effects of corticoids in a subject in need thereof, comprising administering to the subject an effective amount of an NAD⁺ agonist.

Alternatively, a fifth aspect provides use of an NAD⁺ agonist in the manufacture of a medicament for reducing the side effects of corticoids in a subject in need thereof, or an NAD⁺ agonist for use in reducing the side effects of corticoids in a subject in need thereof.

A sixth aspect provides reduction of the side effects of anti-retroviral treatment in a subject in need thereof, comprising administering to the subject an effective amount of an NAD⁺ agonist.

Alternatively, a sixth aspect provides use of an NAD⁺ agonist in the manufacture of a medicament for reducing the side effects of anti-retroviral therapy in a subject in need thereof, or an NAD⁺ agonist for use in reducing the side effects of anti-retroviral therapy in a subject in need thereof.

A seventh aspect provides reduction of the side effects of, or treating or preventing cellular senescence or preventing induction of SASP associated with, PPARγ agonists in a subject in need thereof, comprising administering to the subject an effective amount of an NAD⁺ agonist.

Alternatively, a sixth aspect provides use of an NAD⁺ agonist in the manufacture of a medicament for reducing the side effects of, or treating or preventing cellular senescence or induction of SASP associated with, PPARγ agonists in a subject in need thereof, or an NAD⁺ agonist for use in reducing the side effects of, or treating or preventing cellular senescence or induction of SASP associated with, PPARγ agonists in a subject in need thereof.

A seventh aspect provides treating or preventing cellular senescence or induction of SASP associated with increased caloric intake and obesity in a subject in need thereof, comprising administering to the subject an effective amount of an NAD⁺ agonist.

Alternatively, a seventh aspect provides use of an NAD⁺ agonist in the manufacture of a medicament for treating or preventing cellular senescence associated with, or induction of SASP associated with, increased caloric intake and obesity in a subject in need thereof, or an NAD⁺ agonist for use in treating or preventing cellular senescence associated with, or induction of SASP associated with, increased caloric intake and obesity in a subject in need thereof.

An eighth aspect provides reduction of cellular hypertrophy associated with cellular senescence or induction of SASP in a subject in need thereof, comprising administering to the subject an effective amount of an NAD⁺ agonist.

Alternatively, an eighth aspect provides use of an NAD⁺ agonist in the manufacture of a medicament for reducing cellular hypertrophy associated with cellular senescence or induction of SASP in a subject in need thereof, or an NAD⁺ agonist for use in reducing cellular hypertrophy associated with cellular senescence or induction of SASP in a subject in need thereof.

A ninth aspect provides a pharmaceutical composition for treating or preventing DNA damage in a cell or cellular senescence of a cell or induction of SASP in a cell, comprising an NAD⁺ agonist, and a pharmaceutically acceptable carrier.

A tenth aspect provides a pharmaceutical composition when used for treating or preventing DNA damage in a cell or cellular senescence of a cell or induction of SASP in a cell, or for treating or preventing the effects of aging, or for preventing or treating cellular senescence and/or induction of SASP associated with high caloric intake, obesity, aging or cell stress, or for reducing the side effects of chemotherapy, radiotherapy, corticoid treatment, anti-retroviral treatment, or PPARγ agonist treatment, the composition comprising an NAD⁺ agonist, and a pharmaceutically acceptable carrier.

An eleventh aspect provides a cosmetic formulation comprising an NAD⁺ agonist, and a dermatologically acceptable carrier.

A twelfth aspect provides a kit for treating or preventing DNA damage in a cell or cellular senescence of a cell or induction of SASP in a cell, or for treating or preventing the effects of aging, or for preventing or treating cellular senescence and/or induction of SASP associated with high caloric intake, obesity, aging or cell stress, or for reducing the side effects of chemotherapy, radiotherapy, corticoid treatment, anti-retroviral treatment, and/or PPARγ agonist treatment, the kit comprising an NAD⁺ agonist.

A thirteenth aspect provides a pharmaceutical composition when used for treating or preventing cellular senescence of an adipose cell or induction of SASP in an adipose cell, the composition comprising an NAD⁺ agonist and a pharmaceutically acceptable carrier. In various embodiments, the pharmaceutical composition is used for treating or preventing the effects of aging, or for preventing or treating cellular senescence and/or induction of SASP associated with high caloric intake, obesity, aging or cell stress, or for reducing the side effects of chemotherapy, radiotherapy, corticoid treatment, anti-retroviral treatment, or PPARγ agonist treatment.

### Brief Description of the Drawings

Figure 1 is a photopraph showing A: Senescence associated β-galactosidase staining of white adipose tissue whole mounts (epididymal depot) from 18 month old C57BL6 mice after treatment with or without the NAD⁺ agonist apigenin; and B: comparison of senescence associated β-galactosidase staining of white adipose tissue whole mounts obtained from 18 month and 3 month old mice treated with or without the NAD⁺ agonist apigenin.
Figure 2 is a graph showing the results of quantitative RTPCR analysis of mRNA expression of secreted protein markers of senescence in 6 month old mice on chow or high fat diet (HFD) treated with or without the NAD⁺ agonist apigenin.
Figure 3 is a graph showing the results of quantitative PCR analysis of mRNA expression of biomarkers of senescence in 12 month old mice on chow or high fat diet (HFD) treated with or without the NAD⁺ agonist apigenin.
Figure 4 is a series of bioluminescent images of p16^{LUC} mice treated with either vehicle control (ctrl), Imiquimod (IMQ), or Imiquimod and NMN, followed by ip injection of D-luciferin and bioluminescence imaging.
Figure 5 is a series of bioluminescent images of p16^{LUC} mice treated with either vehicle control, apigenin, doxorubicin or doxorubicin and apigenin, followed by ip injection of D-luciferin and bioluminescence/CT imaging.
Figure 6 is a graph showing: A. blood glucose levels of Sprague-Dawley rats injected daily over 3 days with saline vehicle (ctrl), dexamethasone sodium phosphate (0.8 mg/kg)(dexamethasone), or co-administration of dexamethasone sodium phosphate (0.8mg/kg) and NMN (1.5g/kg) (dexamethasone+NMN); and B. free water as assessed by echoMRI 24 hours after infection of saline vehicle (ctrl), dexamethasone sodium phosphate (0.8 mg/kg)(dexamethasone); or co-administration of dexamethasone sodium phosphate (0.8mg/kg) and NMN (1.5g/kg) (dexamethasone+NMN).

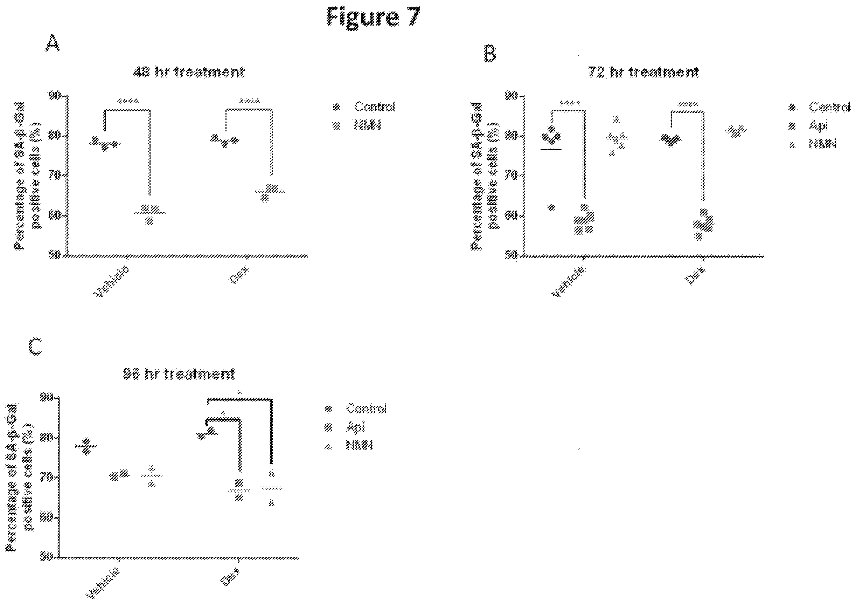

Figure 7 is a graph showing the percentage of SA-β-Gal positive cells as determined using the fluorogenic substrate, C₁₂FDG, by flow cytometry (protocol of Debacq-Chainiaux et al Nature Protocols 2009) in: (A) Cells treated for 48 hours with either dexamethasone (0.1ug/ml) or an ethanol vehicle control, with or without NMN (1mM), n =3; B. cells treated with a pharmaceutical grade dexamethasone sodium phosphate (250nM) with a PBS vehicle control in the presence or absence of NMN (1mM) or apigenin (25uM) for 72 hours, n = 5-6 and C. 96 hours, n = 2. All data are presented as the mean value. * p < 0.05, **** p <0.0001, using two-way ANOVA multiple comparisons test.
Figure 8 is a graph showing intracellular lipid content (as determined by oil red O absorbance) of 3T3-L1 adipocytes treated for 24 hr with the HIV antiretroviral agent stavudine (10 nM) in the presence or absence of NMN (1 mM). Error bars: SD.
Figure 9 is a graph showing showing percentage of intracellular lipid content of 3T3-L1 adipocytes treated for 24 hr with HIV antiretroviral agents (30 µM)as indicated, in the presence or absence of NMN (1 mM) or apigenin (25 µM). Intracellular lipid content was assessed using oil red o staining, which was quantified by reading absorbance at 544 nm.
Figure 10 is a graph showing the changes in body weight of 3-4 month old female C57L6 mice following exposure to 500cGy of whole body γ-irradiation followed by a diet of chow (irradiation), chow supplemented with apigenin (irradiation + apigenin); or following no radiation exposure followed by a diet of chow (control) or chow and apigenin (Apigenin). *p<0.05, **p<0.01, irradiated vs irradiated plus apigenin, 2 way ANOVA, kruskal-wallis multiple comparison test.
Figure 11 is a graph showing the changes in body weight of male C57BL6 mice fed control chow diet supplemented with apigenin (500mg/kg in food) following intraperitoneal administration of doxorubicin (10mg/kg of body weight) or saline/DMSO control.

### Detailed Description of the Invention

Described herein is the treatment or prevention of cellular senescence of a cell. As used herein, "cellular senescence" refers to a condition of a cell in which the cell is viable but has lost the ability to proliferate. Typically, the cell is metabolically active. Cellular senescence may increase with age or exposure to factors that induce DNA damage, such as mutation or chromosomal damage, or that induces a DNA damage response or disruption of chromatin structure resulting in changes in gene expression, such as genes associated with SASP. Senescence is thought to be a result of DNA or chromosomal insults including telomere shortening, chromosomal aneuploidy, DNA strand breaks, DNA chemical modification (e.g. alkylation) and triggering of a DNA damage response. If the DNA damage exceeds a certain threshold, cells are destined to undergo either apoptosis and death, or cellular senescence. Cellular senescence may be caused by, for example, DNA damaging compounds such as chemotherapeutic agents, or DNA damaging radiation such as ionizing and UV radiation.
Senescence may be caused by various other treatment regimes, such as corticoid treatment, anti-retroviral treatment, treatment with PPARγ agonists, treatment with xanthine oxidase inhibitors, treatment with bisphosphonates, treatment with antiprotozoal agents, and treatment with inflammatory agents. Senescence may also be caused by metabolic factors such as increased caloric intake, obesity, insulin resistance, type II diabetes, hyperinsulinaemia, high fat diets, high protein diets, and alterations in gut microbiota associated with these diseases. Cellular senescence may be induced in cells through induction of the senescence associated secretory phenotype (SASP). The senescence associated secretory phenotype refers to senescence induced in otherwise healthy cells through the secretion of proteins, such as pro-inflammatory cytokines, by senescent cells.

In various embodiments, the cellular senescence is caused by:
(a) Ageing of the cell;
(b) DNA damage to the cell;
(c) Contacting the cell with a chemotherapeutic agent;
(d) Irradiating the cell with DNA damaging radiation;
(e) contacting the cell with an anti-retroviral agent;
(f) contacting the cell with a PPARγ agonist;
(g) contacting the cell with a proinflammatory agent;
(h) contacting the cell with a DNA damaging agent;
(i) contacting the cell with an agent that disrupts chromatin structure;
(j) contacting the cell with a corticoid.

As used herein, "DNA damage" refers to changes to the DNA, including changes to one or more chromosomes, of a cell which results in cellular dysfunction. Typically, the DNA damage will induce a DNA damage response in the cell. DNA damage includes, for example, DNA strand breaks, telomere shortening, chemical modification of the DNA (e.g. alkylation), chromosomal rearrangements, and chromosomal aneuploidy, such as chromosomal aneuploidy resulting from uneven chromosome separation during mitosis. In one embodiment, the DNA damage induces cellular senescence.

Cellular senescence occurs, for example, during old age, following exposure to DNA damaging chemicals or radiation (e.g. sunlight, UV light, radiotherapy), or following increased caloric intake and metabolic disease.

Cells that have undergone cellular senescence may exhibit one or more of the following characteristics: formation of γ-H2AX (a phosphorylated form of the histone variant H2AX) nuclear foci; a rise in the level of 53BP1; a rise in the level of NBS1; a rise in the expression and/or level of MDC1; a rise in the expression and/or level of p16^{INK4A}; a rise in the expression and/or level of p21^{CIP1}; a rise in the level of p15^{INK4B}; increased activity of senescence associatedp-galactosidase (SA-β-Gal); production of DNA senescence-associated heterochromatic foci (SAHF); loss of proliferation; a rise in the levels of DEC 1; a rise in the level of DCR 2; trimethylation of histone 3 lysine 9 (H3K9me3); endoplasmic reticulum stress and induction of the unfolded protein response; increased glucose consumption; increased expression and/or secretion of pro-inflammatory cytokines, often termed the "senescence associated secretory phenotype" (SASP), which may include, but is not limited to, PAI 1, IL-6, IL-7, IL-1a, IL-1b, IL-13, IL-15, IL-8, MCP-2, MCP4, MIP-1a, MIP-3a, eotaxin-3, amphiregulin, epiregulin, heregulin, GM-CSF, MIF, EGF, FGF, HGF, VEGF, KGF, PIGH, NGF, MMP1, MMP3, MMP10, MMP12, MMP13, MMP14, IGFBP2, IGFBP3, IGFBP4, IGFBP6, IGFBP7, fibronectin, cathepsin B, TIMP-2; lack of expression of Ki-67; cell enlargement; persistent DNA damage response (DDR) signaling; and formation of DNA segments with chromatin alterations reinforcing senescence (DNA-SCARS), which are nuclear foci which may contain DDR proteins such as phospho-ATM and ATR substrates. Cells that have undergone cellular senescence typically have increased levels of p16^{INK4a} expression relative to the level of P16^{INK4a} expression in cells that have not undergone cellular senescence. Cells that have undergone cellular senescence typically have increased levels of SA-β-Galactivity relative to that of cells that have not undergone cellular senescence.

The inventors have found that agents that raise NAD⁺ levels in a cell are capable of treating or preventing DNA damage, treating or preventing cellular senescence, and preventing secretion of proteins (typically pro-inflammatory cytokines) that are released during cellular senescence. The inventors have found that by raising NAD⁺ levels in the cell, DNA damage or cellular senescence can be prevented or reversed and induction of SASP can be prevented. The inventors further consider that agents that increase the ratio of NAD⁺ to NADH or increase the rate of production of NAD⁺ in a cell will also be effective in treating or preventing DNA damage, treating or preventing cellular senescence and treating or preventing induction of SASP.

As used herein, an "NAD⁺ agonist" (or "NAD⁺ promoter") is an agent which raises NAD⁺ levels in a cell, and/or increases the ratio of NAD⁺ to NADH in a cell, and/or increases production of NAD⁺ in a cell.

In one embodiment, the NAD⁺ agonist is an agent which raises NAD⁺ levels in a cell. An agent which raises NAD⁺ levels in a cell increases the amount of NAD⁺ in the cell relative to the amount of NAD⁺ in the cell prior to contact with the agent. In some embodiments, the agent increases the amount of NAD⁺ in a compartment of the cell. The compartment of the cell may be, for example, the cytosol, the nucleus, mitochondria.

In one embodiment, the NAD⁺ agonist is an agent which increases the ratio of NAD⁺ to NADH in a cell. An agent which raises the ratio of NAD⁺ to NADH in a cell increases the ratio of NAD⁺ to NADH in the cell relative to the ratio of NAD⁺ to NADH in the cell prior to contact with the agent. In some embodiments, the agent increases the ratio of NAD⁺ to NADH in a compartment of the cell. The compartment of the cell may be, for example, the cytosol, the nucleus, mitochondria.

In one embodiment, the NAD⁺ agonist is an agent which increases production of NAD⁺ in a cell. An agent which increases production of NAD⁺ in a cell increases the production of NAD⁺ in the cell relative to the production of NAD⁺ in the cell prior to contact with the agent. In some embodiments, the agent increases the production of NAD⁺ in a compartment of the cell. The compartment of the cell may be, for example, the cytosol, the nucleus, mitochondria.

In one embodiment, the NAD⁺ agonist raises NAD⁺ levels in a cell and increases the ratio of NAD⁺ to NADH in a cell. In one embodiment, the NAD⁺ agonist raises NAD⁺ levels in a cell, increases the ratio of NAD⁺ to NADH in a cell and increases the rate of production of NAD⁺ in the cell.

In one embodiment, the present invention provides a method of treating or preventing cellular senescence of a cell, comprising contacting the cell with an NAD⁺ agonist.

The present invention also provides a method of treating or preventing DNA damage in a cell, comprising contacting the cell with an NAD⁺ agonist.

The present invention also provides a method of treating or preventing induction of SASP in a cell, comprising contacting the cell with an NAD⁺ agonist.

Methods for determining the amount of NAD⁺ in a cell, the ratio of NAD⁺ to NADH in a cell, and the production of NAD⁺ in a cell, are known in the art and are described in, for example, Schwartz et al. (1974) J. Biol. Chem. 249:4138-4143; Sauve and Schramm (2003) Biochemistry 42(31):9249-9256; Yamada et al. (2006) Analytical Biochemistry 352:282-285, or can be determined using commercially available kits such as, for example, NAD/NADH-Glo Assay (Promega Inc.) or NAD/NADH Quantitation Colorimetric Kit (BioVision Inc.).

The cell may be any cell of a subject. In some embodiments, the cell is a neuronal cell. In some embodiments, the cell is a non-neuronal cell.

In one embodiment, the cell is a cell of skin, subcutaneous tissue, fat tissue, muscle tissue, heart tissue, liver tissue, kidney, pancreas, intestine, stomach, or blood vessels. In one embodiment, the cell is an epithelial cell or precursor thereof, endothelial cell or precursor thereof, adipocyte or precursor thereof, fibroblast or precursor thereof, muscle cell or precursor thereof, hepatic cell or precursor thereof, renal cell or precursor thereof, or an immune cell or precursor thereof.

As used herein, the expression "contacting the cell with an NAD⁺ agonist" refers to bringing the NAD⁺ agonist into contact with the cell, or introducing the NAD⁺ agonist into the cell, such that the NAD⁺ levels in the cell are increased.

The NAD⁺ agent may be contacted with the cell by any means which brings the NAD⁺ agonist into contact with the cell or introduces the NAD⁺ agonist into the cell. The NAD⁺ agonist may be contacted with the cell by introducing the NAD⁺ agonist into a tissue containing the cell or administering the NAD⁺ agonist to a subject containing the cell.

In one form, the NAD⁺ agonist may be an agent which reduces breakdown of NAD⁺ in the cell thereby raising the NAD⁺ levels in the cell. An example of an agent which reduces the breakdown of NAD⁺ in the cell is a CD38 inhibitor. CD38 is an enzyme which catalyzes the synthesis and hydrolysis of cyclic ADP-ribose from NAD⁺ and ADP-ribose. CD38 reduces NAD⁺ levels in the cell by converting NAD⁺ to cyclic ADP-ribose. The inventors have found that inhibition of CD38 in the cell results in an increase in NAD⁺ levels and a reduction in cellular senescence. Thus, in one embodiment, the NAD⁺ agonist is a CD38 inhibitor.

As used herein, a "CD38 inhibitor" is an agent which reduces or eliminates the biological activity of CD38. The biological activity of CD38 may be reduced or eliminated by inhibiting enzyme function, or by inhibiting expression of CD38 at the level of gene expression and enzyme production. "Inhibiting" is intended to refer to reducing or eliminating, and contemplates both partial and complete reduction or elimination.

In one embodiment, the CD38inhibitor is an inhibitor of CD38 enzyme function. An inhibitor of CD38 enzyme function is an agent that blocks or reduces the enzymatic activity of CD38.

In one embodiment, the inhibitor of CD38 enzyme function is a compound of formula I: 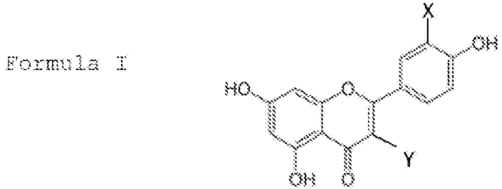 wherein:
X is H or OH; and
Y is H or OH;
or a pharmaceutically acceptable salt, derivative or prodrug thereof.
In one embodiment, X and Y are both H.

An example of an inhibitor of CD38 enzyme function is apigenin, or a pharmaceutically acceptable salt, derivative or prodrug thereof. Apigenin (5,7-dihydroxy-2-(4-hydroxyphenyl)-4H-1-benzopyran-4-one), also known as 4',5,7-trihydroxyflavone, is an isoflavone found in plants, including fruits and vegetables, such as parsley, celery and chamomile. Apigenin has the following structure:

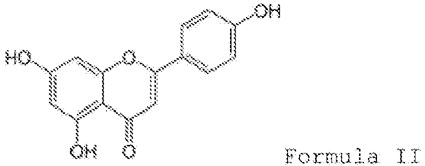

Another example of an inhibitor of CD38 enzyme function is quercetin, or a pharmaceutically acceptable salt, derivative: or prodrug thereof. Quercetin [2-(3,4-dihydroxyphenyl)-3,5,7-trihydroxy-4H-chromen-4-one]) is an isoflavone found in plants, indluding fruits, vegetables, leaves and grains. Queircetin has the following structure:

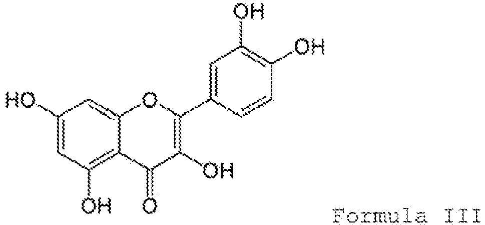

Both apigenin and quercetin have been shown to be inhibitors of CD38 activity in vitro (Esande et al. (2013)Diabetes, 1084-1093).

Isoflavones (such as apigenin or quercetin) are typically administered in isolated form. By "isolated" is meant that the isoflavone has undergone: at least one purification step. When the inhibitor of CD38 enzyme: function is an isoflavone, the inhibitor is conveniently administered in a composition comprising at least 10% w/v inhibitor, at least 20% w/v inhibitor, at least 30% w/v inhibitor, at least 40% w/v inhibitor, at least 50% w/v inhibitor, at least 60% w/v inhibitor, at least 70% w/v inhibitor, at least 80% w/y inhibitor, at least 90% w/v inhibitor at least 95% w/v inhibitor, at least 98% w/v inhibitor. In one embodiment, the inhibitor is in a biologically pure form. Methods for isolation of biologically pure forms of isoflavones such as apigenin and quercetin are known in the art. Biologically pure apigenin and quercetin is also commercially available from, for example, Sigma Chemical Company (St. Louis) (Cat. No. A3145 and Cat. No. Q4951), or Indofine Chemical Company (Cat. No. A-002).

In some embodiments, the CD38 inhibitor is a pharmaceutically acceptable salt, or pro-drug form of the inhibitor of CD38 enzyme function, such as a pharmaceutically acceptable salt or prodrug of apigenin or quercetin. The term "prodrug" is used herein in its broadest sense to include those compounds which are converted *in vivo* to the active form of the drug. Use of the prodrug strategy may optimise the delivery of the NAD⁺ agonist to its site of action.

In one embodiment, the pro-drug of the inhibitor of CD38 enzyme function is an ester or an imine of the inhibitor.

In one embodiment, the NAD⁺ agonist is apigenin.

In another embodiment, the CD38 inhibitor is an inhibitor of CD38 gene expression or enzyme production. An inhibitor of CD38 gene expression or enzyme production is an agent that blocks or reduces transcription or translation of the CD38 gene. Inhibition of CD38 gene expression or enzyme production may be, for example, by RNA interference (RNAi) (e.g. siRNA, shRNA), antisense nucleic acid, locked nucleic acid (LNA), DNAzymes, or ribozymes, which target CD38 mRNA transcripts, by genome editing technologies such as Zinc finger nuceases (ZFN), Transcription Activator-Like effector Nucleases (TALENS), Clustered regular Interspaced Short Palindromic Repeats (CRISPR), or engineered meganuclease reengineered homing nuclease, which target the CD38 gene. "RNAi" refers to a nucleic acid that forms a double stranded RNA, which double stranded RNA has the ability to reduce or inhibit expression of a target gene when the siRNA is present in the same cell as the gene or target gene. "shRNA" or "short hairpin RNA" refers to a nucleic acid that forms a double stranded RNA with a tight hairpin loop, which has the ability to reduce or inhibit expression of a gene or target gene. An "antisense" polynucleotide is a polynucleotide that is substantially complementary to a target polynucleotide and has the ability to specifically hybridize to the target polynucleotide to decrease expression of a target gene. Ribozymes and DNAzymes are catalytic RNA and DNA molecules, respectively, which hybridise to and cleave a target sequence to thereby reduce or inhibit expression of the target gene. General methods of using antisense, ribozyme, DNAzyme and RNAi technology, to control gene expression, are known in the art. Genome editing uses artificially engineered nucleases to create specifc double strand breaks at desired locations in the genome, and harnesses the cells endogenous mechanisms to repair the breaks. Methods for silencing genes using genome editing technologies are described in, for example, Tan et al. (2012) Precision editing of large animal genomes, Adv. Genet. 80: 37-97; de Souza (2011) Primer: Genome editing with engineered nucleases, Nat. Meth. 9(1) 27-27; Smith et al. (2006) A combinatorial approach to create artificial homing endonucleases cleaving chosen sequences, Nucleic Acids Research 34: 22, e149; Umov et al. (2010) Nat. Rev. Genet. 11(9): 636-646. Inhibition of CD38 expression using iRNA is described in, for example, Escande et al. (2013) Diabetes, 62: 1084-1093.

In another embodiment, the NAD⁺ agonist is an agent which promotes synthesis of NAD⁺ in the cell thereby raising NAD⁺ levels in the cell. An example of an agent which promotes synthesis of NAD⁺ is an NAD⁺ precursor. As used herein, an "NAD⁺ precursor" is an intermediate of NAD⁺ synthesis which does not inhibit sirtuin activity. Examples of NAD⁺ precursors include nicotinamide mononucleotide (NMN), nicotinamide riboside (NR), nicotinic acid mononucleotide (NaMN), nicotinic acid adenine dinucleotide (NaAD), 5-phospho-α-D-ribosyl-1-pyrophosphate (PPRP) or a pharmaceutically acceptable salt, derivative or prodrug thereof.

In one embodiment, the NAD⁺ agonist is NMN or a pharmaceutically acceptable salt, derivative or prodrug thereof.

In one embodiment, the NAD⁺ agonist is NR or a pharmaceutically acceptable salt, derivative or prodrug thereof. Examples of derivatives of NR and methods for their production, are described in, for example, US patent no. 8,106,184, the contents of which are incorporated herein by reference.

In another embodiment, the NAD⁺ agonist is a cell permeable form of NAD⁺, derivative or prodrug thereof.

In another embodiment, NAD⁺ levels may be raised by reducing inhibition of translation of the NAD⁺ biosynthetic enzymes NAMPT, NMNAT1, NMNAT2, and NMNAT3. Inhibition of translation of the NAD⁺ biosynthetic enzymes NAMPT, NMNAT1, NMNAT2, and NMNAT3 is mediated by endogenous micro RNA (miRNA) that target NAMPT, NMNAT1, NMNAT2, and NMNAT3. Thus, NAD⁺ levels may be raised in the cell by inhibiting the activity of endogenous miRNA which targets NAMPT, NMNAT1, NMNAT2, and NMNAT3. Accordingly, in one embodiment, the NAD⁺ agonist is an NAMPT, NMNAT1, NMNAT2, and/or NMNAT3 miRNA antagonist. As used herein, a "NAMPT, NMNAT1, NMNAT2, and/or NMNAT3 miRNA antagonist" is an agent which inhibits the activity of miRNA, that inhibits translation of any one or more of NAMPT, NMNAT1, NMNAT2, and NMNAT3. The NAMPT, NMNAT1, NMNAT2, and/or NMNAT3 miRNA antagonist may act by inhibiting NAMPT, NMNAT1, NMNAT2, and/or NMNAT3 miRNA through, for example, RNA interference (RNAi) (e.g. siRNA, shRNA), antisense nucleic acid, locked nucleic acid (LNA), DNAzymes, or ribozymes, which target miRNAs that target NAMPT, NMNAT1, NMNAT2, and/or NHNAT3, or by genome editing technologies such as Zinc finger nuceases (ZFN), Transcription Activator-Like effector Nucleases (TALENS), Clustered regular Interspaced Short Palindromic Repeats: (CRISPR), or engineered meganuclease reengineered homing nuclease, which target the DNA sequences which encode the miRNAs that target NAMPT, NMNAT1, NMNAT2, and/or NMNAT3. Activation domains may be targeted to the genes of NAD biosynthetic genes (e.g. NAMPT, NMNAT1, NMNAT2, and/or NMNAT3) to increase gene expression using CRISPR-directed heterologous regulatory domains (e.g. VP16 or VP64).

In another embodiments NAD⁺ levels may be: raised by contacting the cell with an NAD⁺ agonist which enhances the enzymatic activity of NAD⁺ biosynthetic enzymes, such as the NAD⁺ biosynthetic enzymes NAMPT, NMNAT1, NMNAT2, and/or NMNAT3 or PNC1 from other species such as yeast, flies or plants. For example, P7C3 enhances activity of NAMPT in vitro, thereby increasing the level of intracellular NAD⁺ (Wang et al. (2014) Cell, 158(6):1324-1334). P7C3 has the following structure:

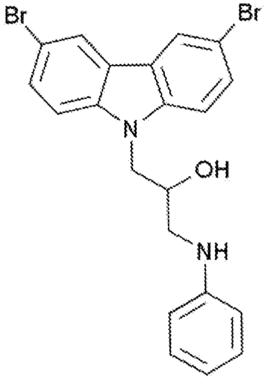

In one embodiment, the agent which raises the NAP⁺ level of the cell is an agent which increases the ratio of NAP⁺ to NADH in the: cell relative to the ratio of NAD⁺ to NADH in the cell prior to contact with the agent. For example, the ratio of the amount of NAD⁺ to NADH may be increased by contacting the cell with an NAD⁺ agonist which activates an enzyme that converts NADH to NAD⁺. For example, β-lapachone (3,4-dihydro-2,2-dimethyl-2H-napthol[1,2-b]pyran-5,6-dione) activates the enzyme NADH:quinone oxidoreductase (NQ01) which catalyses the reduction of quinones to hydroquinones by utilizing NADH as an electron donor, with a consequent increase in the ratio of NAD⁺ to NADH.

As used herein, "treating" means affecting a subject, tissue or cell to obtain a desired pharmacological and/or physiological effect and includes inhibiting the condition, i.e. arresting its development; or relieving or ameliorating the effects of the condition i.e., cause reversal or regression of the effects of the condition. As used herein, "preventing" means preventing a condition from occurring in a cell or subject that may be at risk of having the condition, but does not necessarily mean that condition will not eventually develop, or that a subject will not eventually develop a condition. Preventing includes delaying the onset of a condition in a cell or subject. In one embodiment, treating achieves the result of reversing cellular senescence in the recipient subject. In one embodiment, preventing achieves the result of preventing the onset of cellular senescence in a recipient subject.

In one embodiment, treating or preventing cellular senescence in the cell of a subject comprises comprises contacting the NAD⁺ agonist with the cell of the subject by administering to the subject an effective amount of the NAD⁺ agonist. An "effective amount" is an amount of an agent that will elicit the biological or medical response of a cell, tissue, system, animal or human that is being sought by the researcher, veterinarian, medical doctor or other clinician.

As used herein, the term "subject" refers to a mammal. The mammal may, for example, be a human, primate, livestock animal (e.g. sheep, cow, horse, donkey, pig), companion animal (e.g. dog, cat), laboratory test animal (e.g. mouse, rabbit, rat, guinea pig, hamster), captive wild animal (e.g. fox, deer). Typically the mammal is a human or primate. More typically, the mammal is a human. Although the present invention is exemplified using a murine model, the method of the present invention may be applied to other species, in particular humans.

The NAD⁺ agonist may be administered as a pharmaceutical composition comprising the NAD⁺ agonist, and a pharmaceutically acceptable carrier. A "pharmaceutically acceptable carrier" is a carrier that it is compatible with the other ingredients of the composition and is not deleterious to a subject. The compositions may contain other therapeutic agents as described below, and may be formulated, for example, by employing conventional solid or liquid vehicles or diluents, as well as pharmaceutical additives of a type appropriate to the mode of desired administration (for example, excipients, binders, preservatives, stabilizers, flavours, etc.) according to techniques such as those well known in the art of pharmaceutical formulation (See, for example, Remington: The Science and Practice of Pharmacy, 21st Ed., 2005, Lippincott Williams & Wilkins).

The NAD⁺ agonist may be administered by any means which brings the NAD⁺ agent into contact with the cell. The NAD⁺ agonist may, for example, be administered orally, such as in the form of tablets, capsules, granules or powders; sublingually; buccally; parenterally, such as by subcutaneous, intravenous, intramuscular, intra(trans)dermal, intraperitoneal, or intracisternal injection or infusion techniques (e.g., as sterile injectable aqueous or nonaqueous solutions or suspensions); nasally such as by inhalation spray or insufflation; topically, such as in the form of a cream or ointment; ocularly in the form of a solution or suspension; vaginally in the form of pessaries, tampons or creams; or rectally such as in the form of suppositories; in dosage unit formulations containing non-toxic, pharmaceutically acceptable vehicles or diluents. The NAD⁺ agonist may, for example, be administered in a form suitable for immediate release or extended release. Immediate release or extended release may be achieved by the use of suitable pharmaceutical compositions comprising the NAD⁺ agonist, or, particularly in the case of extended release, by the use of devices such as subcutaneous implants.

The pharmaceutical compositions for administration may conveniently be presented in dosage unit form and may be prepared by any of the methods well known in the art of pharmacy. These methods generally include the step of bringing the agent into association with the carrier which constitutes one or more accessory ingredients. In general, the pharmaceutical compositions are prepared by uniformly and intimately bringing the compound into association with a liquid carrier or a finely divided solid carrier or both, and then, if necessary, shaping the product into the desired formulation. In the pharmaceutical composition the active compound is included in an amount sufficient to produce the desired effect. As used herein, the term "composition" is intended to encompass a product comprising the specified ingredients in the specified amounts, as well as any product which results, directly or indirectly, from combination of the specified ingredients in the specified amounts.

The pharmaceutical compositions may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents such as sweetening agents, flavouring agents, colouring agents and preserving agents, e.g. to provide pharmaceutically stable and palatable preparations. Tablets containing the NAD⁺ agonist, may be prepared in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated to form osmotic therapeutic tablets for control release.

Formulations for oral use may also be presented as hard gelatin capsules wherein the NAD⁺ agonist is mixed with an inert solid diluent, for example, calcium carbonate, calcium phosphate or kaolin, or as soft gelatin capsules wherein the agent is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the NAD⁺ agonist in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the compound in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions may also be in the form of oil-in-water emulsions. The oily phase may be a vegetable oil, for example olive oil or arachis oil, or a mineral oil, for example liquid paraffin or mixtures of these. Suitable emulsifying agents may be naturally- occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavouring and colouring agents.

The pharmaceutical compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectable formulations.

The NAD⁺ agonist can also be administered in the form of liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolisable lipid capable of forming liposomes can be used. The present compositions in liposome form can contain, in addition to a compound of the present invention, stabilizers, preservatives, excipients and the like. The preferred lipids are the phospholipids and phosphatidyl cholines, both natural and synthetic. Methods to form liposomes are known in the art.

The pharmaceutical composition may further comprise other therapeutically active compounds, including other therapeutically active compounds referred to herein or other therapeutically active compounds which are usually applied in the treatment of conditions referred to herein. Selection of the appropriate agents for use in combination therapy may be made by one of ordinary skill in the art, according to conventional pharmaceutical principles. The combination of therapeutic agents may act synergistically to effect the treatment or prevention of the various disorders described above. Using this approach, one may be able to achieve therapeutic efficacy with lower dosages of each agent, thus reducing the potential for adverse side effects.

The NAD⁺ agonist may be administered topically as a cosmetic formulation comprising the NAD⁺ agonist which raises NAD⁺ levels, and a dermatologically acceptable carrier. A "dermatologically acceptable carrier" is a carrier which is suitable for topical application to the skin. Suitable dermatologically acceptable carriers include: emulsions such as oil-in-water, water-in-oil, oil-in-water-in silicone and water-in-oil-in water emulsions; anhydrous liquid solvents such as oils (e.g. mineral oil), alcohols (e.g. ethanol, isopropanol), silicones (e.g. dimethicone, cyclomethicone); aqueous-based single phase liquid solvents. In addition, the cosmetic formulation may comprise skin actives such as vitamins (e.g. vitamin B3, vitamin E, etc), hydroxy acids (e.g. salicylic acid, glycolic acid); exfoliation agents such as zwitterionic surfactants; sunscreen (e.g. 2-ethylhexyl-p-methoxycinnamate, 4,4'-t-butyl methoxydibenzoyl-methane, octocrylene, , zinc oxide, titanium dioxide); anti-inflammatory agents; anti-oxidants (e.g. tocopherol); metal chelators (e.g. iron chelators); retinoids; depilatory agents; skin lightening agents; anti-microbial agents.

In some embodiments, the cosmetic formulations may be in the form of ointments, pastes, creams, lotions, gels, powders, solutions or patches. In certain embodiments, the formulations and compositions are creams, which may further contain saturated or unsaturated fatty acids such as steaeric acid, palmitic acid, oleic acid, palmato-oleic acid, acetyle, or aryl oleyl alcohols, stearic acid. Creams may also contain a non-ionic surfactant, for example, polyoxy-40-stearate.

Cells that have undergone cellular senescence are unable to replace themselves and contribute to dysfunctional tissues contributing to significant health side effects and loss of quality of life during and post chemotherapy and/or radiotherapy, including, for example, metabolic dysfunction, accelerated aging, and an increased risk of cancers in later life, unrelated to the initial cancer. Side effects during chemotherapy treatment include, for example, pain, neuropathic pain, nausea, metabolic dysfunction, immunosenescence, muscle fatigue, cardiovascular pathologies, and bowel dysfunction. The senescent cells also secrete pro-inflammatory proteins that contribute to health disorders associated with chemotherapy.

The inventors envisage that administration of an effective amount of an NAD⁺ agonist before, after and/or during chemotherapy, may alleviate the side effects of chemotherapy by reducing the number of cells which undergo cellular senescence and/or induction of SASP, or by restoring back to a functional state at least some of those cells which have undergone cellular senescence.

As described in the Examples, the inventors have found that mice administered the chemotherapeutic agent doxorubicin in combination with the NAD⁺ agonist apigenin show reduced levels of p16^{INKa} induction compared to mice administered doxorubicin without apigenin, indicating that administration of an NAD⁺ agonist reduces the senescence inducing effects of doxorubicin.

As described in the Examples, the inventors have also found that the extent of cellular senescence induced by contacting cells with various chemotherapeutic agents is reduced when cells are also contacted with the NAD⁺ agonists NMN or apigenin. In this regard, co-administration to C2C12 myoblasts of NMN or apigenin with each of the chemotherapeutic agents altretamine, aminolevulinic acid, azacitidine, bleomycin sulphate, bortezomib, celecoxib, cisplatin, cyclophosphamide, doxorubicin, etoposide, fluorouracil, gefitinib, gemcitabine, imatinib, mechlorethamine, mercaptopurine, methotrexate, mitotane, oxaliplatin, paclitaxel, pentostatin, procarbizine, raloxifene, romidepsin, sorafenib, tamoxifen, thiotepa, topotecan, tretinoin, vemurafenib, vincristine sulfate, vismodegib, or vorinostat, reduces the extent to which the cells undergo senescence compared to the extent to which the cells undergo senescence when administered each of these chemotherapeutic agents alone.

The inventors also envisage that administration of an effective amount of an NAD⁺ agonist before, after and/or during radiotherapy may alleviate the effects of radiotherapy by reducing the number of cells which undergo cellular senescence and/or induction of SASP, or by restoring back to a functional state at least some of those cells which have undergone cellular senescence.

As described in the Examples, the inventors have found that irradiated mice undergo less weight loss if fed a diet containing the NAD⁺ agonist apigenin when compared to mice on a diet without apigenin, indicating that NAD⁺ agonists can reduce at least some of the side effects of irradiation.

The inventors envisage that administration of an effective amount of an NAD⁺ agonist before, during and/or after irradiation may treat or prevent DNA damage or cellular senescence or induction of SASP associated with irradiation. The NAD⁺ agonist may, for example, be administered to treat or prevent DNA damage or cellular senescence or induction of SASP associated with exposure to UV radiation, α radiation, β radiation, γ radiation, x-ray radiation, cosmic radiation, or combinations thereof. For example, NAD⁺ agonists may be administered to subjects exposed to, or at risk of being exposed to: radiation from medical imaging devices, such as x-rays, PET scans, CT scans; nuclear radiation such as from nuclear fallout or nuclear accidents; UV radiation from sun exposure or other UV sources; cosmic radiation exposure from extended periods spent at high altitudes (e,g. pilots and aeroplane attendants, frequent aeroplane travellers, astronauts); radiotherapy.

The inventors further envisage that administration of an effective amount of an NAD⁺ agonist before, after and/or during a combination of radiotherapy and chemotherapy, may alleviate the effects of combination radiotherapy and chemotherapy by reducing the number of cells which undergo cellular senescence and/or induction of SASP, or by restoring back to a functional state at least some of those cells which have undergone cellular senescence.

The NAD⁺ agonist may, for example, be administered to reduce the side effects of one or more of the following chemotherapeutic agents: mitotic inhibitors, such as vinca alkaloids, including vincristine, vinblastine, vinorelbine, vindesine, vinflunine; podophyllotoxin; taxanes, including docetaxel, larotaxel, ortataxel, paclitaxel, and tesetaxel; epothilones, such as ixabepilone; topoisomerase I inhibitors, such as topotecan, irinotecan, camptothecin, rubitecan, and belotecan; topoisomerase type II inhibitors, including amsacrine, etoposide, etoposide phosphate, and teniposide; anthracyclines, such as aclarubicin, daunorubicin, doxorubicin, epirubicin, idarubicin, amrubicin, pirarubicin, valrubicin, and zorubicin; anthracenediones, such as mitoxantrone and pixantrone; antimetabolites, including dihydrofolate reductase inhibitors, such as aminopterin, methotrexate, pemetrexed; thymidylate synthase inhibitors, such as raltitrexed and pemetrexed; adenosine deaminase inhibitors, including pentostatin, halogenated; or ribonucleotide reductase inhibitors, such as cladribine, clofarabine, and fludarabine; thiopurines, including thioguanine and mercaptopurine; thymidylate synthase inhibitors, including fluorouracil, capecitabine, tegafur, carmofur, and floxuridine; DNA polymerase inhibitors, such as cytarabine; ribonucleotide reductase inhibitors, such as gemcitabine; hypomethylating agents, including azacitidine, and decitabine; and ribonucleotide reductase inhibitors, such as hydroxyurea; cell-cycle nonspecific antineoplastic agents, including alkylating agents such as nitrogen mustards, including mechlorethamine, cyclophosphamide, ifosfamide, trofosfamide, chlorambucil, melphalan, prednimustine, bendamustine, uramustine, estramustine; nitrosoureas, including carmustine, lomustine, semustine, fotemustine; nimustine, ranimustine, and streptozocin; alkyl sulfonates, including busulfan, mannosulfan, and treosulfan; aziridines, including carboquone, thioTEPA, triaziquone, and triethylenemelamine; alkylating-like agents, including platinum agents such as cisplatin, carboplatin, oxaliplatin, nedaplatin, triplatin tetranitrate, satraplatin, hydrazines, such as procarbazine, triazenes, such as dacarbazine, temozolomide, altretamine, and mitobronitol, and streptomycins, such as actinomycin, bleomycin, daunomycin, mitomycin, and plicamycin; photosensitizers, including aminolevulinic acid, methyl aminolevulinate, efaproxiral, and porphyrin derivatives, such as porfiiner sodium, talaporfin, temoporfin, and verteporfin; kinase inhibitors such as sorafenib, bosutinib, neratinib, lapatinib, nilotinib, erlotinib, gefitinib, sunitinib, dasatinib and imatinib; enzyme inhibitors, including farnesyltransferase inhibitors such as tipifarnib, cyclin-dependent kinase inhibitors, such as alvocidib and seliciclib, proteasome inhibitors, such as bortezomib, phosphodiesterase inhibitors, such as anagrelide, IMP dehydrogenase inhibitors, such as tiazofurine, lipoxygenase inhibitors, such as masoprocol, and PARP inhibitors, such as olaparib; receptor antagonists, such as endothelin receptor antagonists including atrasentan, retinoid X receptor antagonists, such as bexarotene, and testolactone; estrogen receptor antagonists, such as tamoxifen; hedgehog inhibitors such as vismodegib; histone deactylase inhibitors such as vorinostat, romidepsin; B-RAF inhibitors such as vemurafenib; estrogen receptor modulators such as raloxifene; and other chemotherapeutics, including mitatane, amsacrine, trabectedin, retinoids such as alitretinoin and tretinoin, arsenic trioxide, asparagine depleters such as asparaginase or pegaspargase, celecoxib, demecolcine, elesclomol, elsamitrucin, etoglucid, and lonidamine. In one embodiment, the chemotherapeutic agent is selected from the group consisting of altretamine, aminolevulinic acid, azacitidine, bleomycin sulphate, bortezomib, celecoxib, cisplatin, cyclophosphamide, doxorubicin, etoposide, fluorouracil, gefitinib, gemcitabine, imatinib, mechlorethamine, mercaptopurine, methotrexate, mitotane, oxaliplatin, paclitaxel, pentostatin, procarbizine, raloxifene, romidepsin, sorafenib, tamoxifen, thiotepa, topotecan, tretinoin, vemurafenib, vincristine sulfate, vismodegib, or vorinostat.

The NAD⁺ agonist may also be administered to reduce the side effects of radiotherapy, either alone or in combination with chemotherapy, including chemotherapy using any one or more of the above chemotherapeutic agents.

Pharmaceutical compositions for reducing the effects of chemotherapy and/or radiotherapy may be administered intravenously, orally, intra-peritoneally, intra-muscularly, subcutaneously, or intradermally. Typically, the pharmaceutical composition comprising the NAD⁺ agonist is formulated in a pharmaceutical composition in which the NAD⁺ agonist is the only active ingredient. However, it is envisaged that in some embodiments, the pharmaceutical composition comprising the NAD⁺ agonist may be formulated with one or more chemotherapeutic agents for simultaneous administration.

In one embodiment, the pharmaceutical composition comprises an NAD⁺ agonist selected from the group consisting of NMN and apigenin, one or more chemotherapeutic agents selected from the group consisting of altretamine, aminolevulinic acid, azacitidine, bleomycin sulphate, bortezomib, celecoxib, cisplatin, cyclophosphamide, doxorubicin, etoposide, fluorouracil, gefitinib, gemcitabine, imatinib, mechlorethamine, mercaptopurine, methotrexate, mitotane, oxaliplatin, paclitaxel, pentostatin, procarbizine, raloxifene, romidepsin, sorafenib, tamoxifen, thiotepa, topotecan, tretinoin, vemurafenib, vincristine sulfate, vismodegib, or vorinostat, and a pharmaceutically acceptable carrier.

In one embodiment, the pharmaceutical composition comprises one more NAD⁺ agonists selected from the group consisting of NMN and apigenin, the chemotherapeutic agent doxorubicin, and a pharmaceutically acceptable carrier.

As described in the Examples, the inventors have found that stavudine induced lipodystrophy of adipocytes can be reduced or prevented by co-administering the NAD⁺ agonist NMN or apigenin. The inventors believe that the use of NAD⁺ agonists to reduce or prevent lipodystrophy of stavudine treated adipocytes can be extended to prevent or reduce age-related lipodystrophy.

In one embodiment, there is provided a pharmaceutical composition comprising one more NAD⁺ agonists selected from the group consisting of NMN and apigenin, and a dermatologically acceptable carrier.

In another embodiment, there is provided a treatment or prevention of DNA damage or cellular senescence or induction of SASP associated with corticoid treatment, such as glucocorticoid treatment, comprising administering to a subject undergoing, or who has undergone, glucocorticoid treatment, an effective amount of an NAD⁺ agonist. Corticoid treatment refers to treatment with one or more corticoids. Examples of corticoids include cortisol, prednisone, prednisolone, dexamethasone, triamcinolone, beclometasone, fludrocortisone, deoxycorticosterone, and aldosterone. Administration of an NAD⁺ agonist may abrogate or alleviate long-term side effects of glucocorticoid treatment including metabolic diseases such as hyperglycaemia and insulin resistance, weight gain, degeneration and/or pathologies of tissues (e.g. tendons), andneurodevelopmental disorders arising from steroid use during pregnancy or in infants.

As described in the Examples, the inventors have found that the extent of cellular senescence induced by contacting cells with dexamethasone is reduced when cells are also contacted with NMN or apigenin. Thus, in various embodiments, there is provided a pharmaceutical composition for treating or preventing DNA damage and/or cellular senescence or induction with SASP associated with dexamethasone treatment comprising:
(a) NMN;
(b) Apigenin;
(c) NMN and apigenin;
(d) NMN and dexamethasone;
(e) apigenin and dexamethasone; or
(f) NMN and apigenin and dexamethasone,
and a pharmaceutically acceptable carrier.

In another embodiment, there is provided a treatment or prevention of DNA damage or cellular senescence or induction of SASP associated with anti-retroviral treatment, comprising administering to a subject undergoing, or who has undergone, antiretroviral treatment, an effective amount of an NAD⁺ agonist. Anti-retroviral treatment refers to treatment with one or more anti-retroviral agents. Examples of anti-retroviral agents include didanosine (2',3'-dideoxyinosine), lamivudine (3TC)(2',3'-dideoxy-3'-thiacytidine), stavudine (2',3'-didehydro-2',3'-dideoxythymidine), zalcitabine (2',3'-dideoxycytidine),zidovudine (AZT) (azidothymidine), delavirdine, loviride, efavirenz ((4S)-6-chloro-4-(2-cyclopropylethynyl)-4-(trifluoromethyl)-2,4-dihydro-1H-3,1-benzoxazin-2-one, lopinavir ((2*S*)-*N*-[(2*S*,4*S*,5*S*)-5-[2-(2,6-dimethylphenoxy)acetamido]-4-hydroxy-1,6-diphenylhexan-2-yl]-3-methyl-2-(2-oxo-1,3-diazinan-1-yl)butanamide), Ritonavir (1,3-thiazol-5-ylmethyl *N*-[(2*S*,3*S*,5*S*)-3-hydroxy-5-[(2*S*)-3-methyl-2-{[methyl({[2-(propan-2-yl)-1,3-thiazol-4-yl]methyl})carbamoyl]amino}butanamido]-1,6-diphenylhexan-2-yl]carbamate), nevirapine ( 11-cyclopropyl-4-methyl-5,11-dihydro-6H-dipyrido[3,2-b:2',3'-e][1,4]diazepin-6-one), invirase (saquinavir)(( 2*S*)-*N*-[(2*S*,3*R*)-4-[(3*S*)-3-(*tert*-butylcarbamoyl)-decahydroisoquinolin-2-yl]-3-hydroxy-1-phenylbutan-2-yl]-2-(quinolin-2-ylformamido)butanediamide), tipranavir (*N*-{3-[(1*R*)-1-[(2*R*)-6-hydroxy-4-oxo-2-(2-phenylethyl)-2-propyl-3,4-dihydro-2H-pyran-5-yl]propyl]phenyl}-5-(trifluoromethyl)pyridine-2-sulfonamide) and tenofovir (tenofovir disoproxil)( ({[(2*R*)-1-(6-amino-9*H*-purin-9-yl)propan-2-yl]oxy}methyl)phosphonic acid). Administration of an NAD⁺ agonist may abrogate or alleviate side effects of anti-retroviral treatment including subcutaneous lipodystrophy, metabolic diseases such as hyperglycaemia and insulin resistance, hyperlipidaemia, renal toxicity, hepatic toxicity, nausea, pain, and gastrointestinal symptoms.

As described in the Examples, the inventors have found that co-administration of NMN or apigenin with the antiretroviral agents stavudine, AZT, 3TC, efavirenz, Lopinavir, Ritonavir to adipocytes reduces the extent of lipodystrophy in 3T3-L1 adipocytes compared to the extent of lipodystrophy in adipocytes treated with the antiretroviral agent alone.

As also described in the Examples, co-administration to C2C12 myoblasts of NMN or apigenin with each of the antiretroviral agents AZT, 3TC, zalcitabine, efavirenze, lopinavir, ritonavir, nevirapine, tipranavir, tenofovir and invirase, reduces the extent to which the cells undergo senescence compared to the extent to which the cells undergo senescence when administered each of the antiretroviral agents alone.

In various embodiments, there is provided a pharmaceutical composition for reducing or preventing lipodystrophy associated with anti-retroviral treatment comprising:
(a) NMN;
(b) Apigenin;
(c) NMN and apigenin;
(d) NMN and one or more antiretroviral agents selected from the groups consisting of Stavudine, A2T, 3TC, Efavirenz, Lopinavir, Ritonavir;
(e) Apegenin and one or more antiretroviral agents selected from the groups consisting of Stavudine, A2T, 3TC, Efavirenz, Lopinavir, and Ritonavir;
(f) NMN and apigenin and one or more antiretroviral agents selected from the group consisting of stavudine, A2T, 3TC, Efavirenz, Lopinavir and Ritonavir,
and a pharmaceutically acceptable carrier.

In one embodiment, the one or more antiretroviral agents selected from the group consisting of Stavudine, A2T, 3TC, Efavirenz, Lopinavir and Ritonavir is Stavudine.

In various embodiments, there is provided a pharmaceutical composition for treating or preventing cellular senescence or induction of SASP associated with anti-retroviral treatment comprising:
(a) NMN;
(b) Apigenin;
(c) NMN and apigenin;
(d) NMN and one or more antiretroviral agents selected from the group consisting of AZT, 3TC, zalcitabine, efavirenze, lopinavir, ritonavir, nevirapine, tipranavir, tenofovir and invirase;
(e) Apigenein and one or more antiretroviral agents selected from the group consisting of AZT, 3TC, zalcitabine, efavirenze, lopinavir, ritonavir, nevirapine, tipranavir, tenofovir and invirase; or
(f) NMN and apigenin and one or more antiretroviral agents selected from the group consisting of AZT, 3TC, zalcitabine, efavirenze, lopinavir, ritonavir, nevirapine, tipranavir, tenofovir and invirase,
and a pharmaceutically acceptable carrier.

In another embodiment, there is provided a method of treating or preventing DNA damage or cellular senescence or induction of SASP associated with PPARyagonists such as thiazolidinediones, comprising administering to a subject undergoing, or who has undergone, PPARγ agonist treatment, an effective amount of an NAD⁺ agonist. PPARγ agonist treatment refers to treatment with one or more PPARγ agonists. Examples of PPARγ agonists include the thiazolidinediones: e.g. pioglitazone, rosiglitazone and troglitazone. Administration of an NAD⁺ agonist may reduce cellular senescence and/or induction of SASP associated with PPARyagonist treatment, which may be associated with disorders including adverse cardiac events, and increased risk of cancer.

As described in the Examples, co-administration to C2C12 myoblasts of NMN or apigenin with the PPARγ agonist rosiglitazone reduces the extent to which the cells undergo senescence compared to the extent cells undergo senescence when administered rosiglitazone alone.

In various embodiments, there is provided a pharmaceutical composition for treating or preventing cellular senescence associated with treatment with Rosiglitazone, comprising:
(i) NMN
(j) Apigenin;
(k) NMN and apigenin;
(l) NMN and rosiglitazone;
(m) apigenin and rosiglitazone; or
(n) NMN and apigenin and rosiglitazone,
and a pharmaceutically acceptable carrier.

In another embodiment, there is provided a method of treating or preventing DNA damage and/or cellular senescence and/or induction of SASP associated with increased caloric intake and/or obesity, comprising administering to a subject with increased caloric intake or obesity, an effective amount of an NAD⁺ agonist. Administration of an NAD⁺ agonist may reduce cellular senescence and/or induction of SASP associated with increased caloric and/or obesity.

In another embodiment, there is provided a method of treating or preventing cellular hypertrophy, and diseases associated with cellular hypertrophy in a subject in need thereof, such as for example cardiac pathologies arising from cardiac hypertrophy, comprising administering to a subject in need thereof an effective amount of an NAD⁺ agonist. Senescent cells maintain constitutive activation of the mammalian target of rapamycin (mTOR) complex, which maintains both proliferation and cell growth. As senescent cells have entered cell cycle arrest, mTOR signaling can no longer cause proliferation, but instead results in growth of existing cells and cellular proliferation. By preventing cellular senescence, cellular hypertrophy and diseases associated with hypertrophy may be prevented or treated.

In another embodiment, there is provided a method of treating or preventing chronic inflammatory diseases in a subject in need thereof. Chronic inflammatory disease may arise through induction of the senescence associated secretory phenotype (SASP) which includes the secretion of pro-inflammatory cytokines that may cause inflammatory disorders.

As described in the Examples, co-administration of NMN and IMQ reduces the inflammatory effects of IMQ and reduced the induction of cellular senescence as determined by p16^{lNK4a} expression in p16^{LUC} mice.

Pharmaceutical compositions for reducing or preventing inflammation associated with treatment with IMQ are typically administered topically. In various embodiments, pharmaceutical compositions for reducing or preventing inflammation associated with treatment with IMQ comprises:
(a) NMN;
(b) Agigenin;
(c) NMN and apigenin;
(d) NMN and IMQ;
(e) apigenine and IMQ; or
(f) NMN, apigenin and IMQ,
and a pharmaceutically acceptable carrier.

In another embodiment, there is provided a method of treating or preventing DNA damage or cellular senescence or induction of SASP associated with treatment with xanthine oxidase inhibitors in a subject, comprising administering to a subject in need thereof an effective amount of an NAD⁺ agonist. Examples of xanthine oxidase inhibitors include allopurinol (1*H*-pyrazolo[3,4-d]pyrimidin-4(2H)-one), oxypurinol (1,2-Dihydropyrazolo[4,3-e]pyrimidine-4,6-dione) and tisopurine (1,2-Dihydro-4H-pyrazolo[3,4-d]pyrimidine-4-thione).

As described in the Examples, co-administration to C2C12 myoblasts of NMN or apigenin with the xanthine oxidase inhibitor allopurinol reduces the extent to which the cells undergo senescence compared to the extent cells undergo senescence when administered allopurinaol alone.

In one embodiment, there is provided a pharmaceutical composition comprising an NAD⁺ agonist selected from the group consisting of NMN and apigenin, a xanthine oxidase inhibitor, and a pharmaceutically acceptable carrier. In one embodiment, the xanthine oxidase inhibitor is allopurinol.

In another embodiment, there is provided a method of treating or preventing DNA damage or cellular senescence or induction of SASP associated with treatment with an antiprotozoal agent in a subject, such as quinacrine (also known as mepacrine)((*RS*)-*N*'-(6-Chloro-2-methoxy-acridin-9-yl)-N,N-diethylpentane-1,4-diamine), comprising administering to a subject in need thereof an effective amount of an NAD⁺ agonist.

As described in the Examples, co-administration to C2C12 myoblasts of NMN or apigenin with the antiprotozoal agent quinacrine reduces the extent to which the cells undergo senescence compared to the extent cells undergo senescence when administered quinacrine alone.

In one embodiment, there is provided a pharmaceutical composition comprising an NAD⁺ agonist selected from the group consisting of NMN and apigenin, quinacrine, and a pharmaceutically acceptable carrier.

In another embodiment, there is provided a method of treating or preventing DNA damage or cellular senescence or induction of SASP associated with treatment with a bisphosphonate in a subject, comprising administering to a subject in need thereof an effective amount of an NAD⁺ agonist. Bisphosphonates are agents that prevent the loss of bone mass, and are therefore used to treat osteoporosis. Examples of bisphosphonates include etidronate, clodronate, tiludronate, pamidronate, neridronate, olpadronate, alendronate, ibandronate, risedronate and zoledronate.

As described in the Examples, co-administration to C2C12 myoblasts of NMN or apigenin with the disphosphonate zolendronate reduces the extent to which the cells undergo senescence compared to the extent cells undergo senescence when administered zolendronate alone.

In one embodiment, there is provided a pharmaceutical composition comprising an NAD⁺ agonist selected from the group consisting of NMN and apigenin, a bisphosponate, and a pharmaceutically acceptable carrier. In one embodiment, the bisphosphonate is zolendronate.

In another embodiment, there is provided a method of preventing induction of the senescence associated secretory phenotype (SASP) which may lead to non-cell autonomous induction of senescence in other cells.

In one embodiment, there is provided a method of treating or preventing the effects of skin aging. As used herein, "skin aging" refers to changes in the skin structure or macromolecular arrangement of skin arising from cellular senescence associated with UV light exposure, sunlight exposure, chemical exposure, physical wear, and/or biological aging. The effects of skin aging include one or more of the following conditions: skin thinning, skin wrinkling (e.g. superficial wrinkles, deep wrinkles, frown lines, expression lines, dermatoheliosis, photodamage, premature skin aging, crevices, bumps), dryness, scaliness, flakiness, roughness, abnormal epidermal differentiation (e.g. keratosis), loss of skin elasticity, loss of skin firmness, loss of skin tightness, sagging, skin discolouration, thinning of the epidermis, and blotching. By topically or orally administering an effective amount of an NAD⁺, agonist, the effects of skin aging may be prevented or reduced. Typically, the NAD⁺ agonist is administered topically.

In one embodiment, skin aging may be treated by applying cosmetic formulations referred to above. In this regard, cosmetic formulations for treating or preventing the effects of skin aging may be topically applied to the areas of the skin to be treated in an amount that is safe and effective. The amount of composition to be applied, the frequency at which the composition is applied, and the period of use, will vary depending on the condition to be treated or prevented, the amount of agent present in the formulation, and the age and health of the subject. In one form, the cosmetic composition is for regular extended use on daily, weekly or monthly basis. Such cosmetic compositions may be in the form of a skin lotion, cream, lipstick, emulsion, spray, foundation, cosmetic, conditioner, etc.

Also provided is an article of manufacture and a kit, comprising a container comprising an NAD⁺ agonist. The container may be simply be a bottle comprising the NAD⁺ agonist in oral dosage form, each dosage form comprising a unit dose of the NAD⁺ agonist. For example, apigenin in an amount for instance from about 100mg to 750mg, or NMN in an amount from about 100mg to 750 mg. The kit will further comprise printed instructions. The article of manufacture will comprise a label or the like, indicating treatment of a subject according to the present method. In one form, the article of manufacture may be a container comprising the NAD⁺ agonist in a form for topical dosage. For example, the NAD⁺ agonist may be in the form of a cream in a disposable container such as a tube or bottle.

The term "administering" should be understood to mean providing a compound to a subject in need of treatment.

It will be understood that the specific dose level and frequency of dosage for any particular subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

In order to exemplify the nature of the present invention such that it may be more clearly understood, the following non-limiting examples are provided.

The present embodiments are, therefore, to be considered in all respects as illustrative and not restrictive.

### Examples

### Example 1

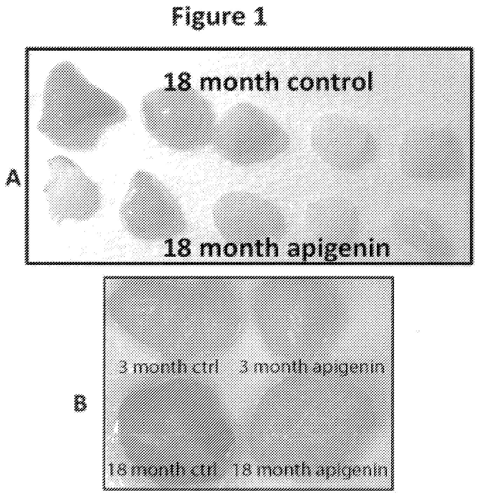

18 month old C57BL6 mice were treated with or without 100 mg/day intraperitoneal apigenin for 7 days. Epidydimal adipose tissue whole mounts were obtained from the mice and stained for expression of senescence associated β-galactosidase according to the chromogenic protocol of Debacq-Chainiaux F et al., Protocols to Detect Senescence-asociated beta-galactosidase (SA-betagal) activity, a biomarker of senescent cells in culture and in vivo, Nature Protocols 2009; 4(12): 1798-1806. Cells that have undergone cellular senescence express β-galactosidase and stain blue (which can be seen as a darker grey in Figure 1A). Figure 1A shows various tissue samples from 18 month old mice fed chow (top row) or chow supplemented daily with 100mg/kg apigenin (bottom row). Blue Staining of tissue samples from mice treated with apigenin was less compared to chow fed mice, indicating that cellular senescence was reduced in mice fed apigenin. Figure 1B shows SA-β-gal staining of adipose tissue samples obtain from 3 old and 18 month old mice fed chow (ctrl) or chow supplemented with apigenin. Staining shows that 18 month old mice fed chow showed the greater staining, while mice fed chow supplementated with apigenin showed little or no staining after 18 months.

As can be seen from Figure 1, adipose tissue from mice fed apigenin had significantly less senescence-associated β-galactosidase expression, and therefore less cellular senescence, than tissue from control mice.

### Example 2

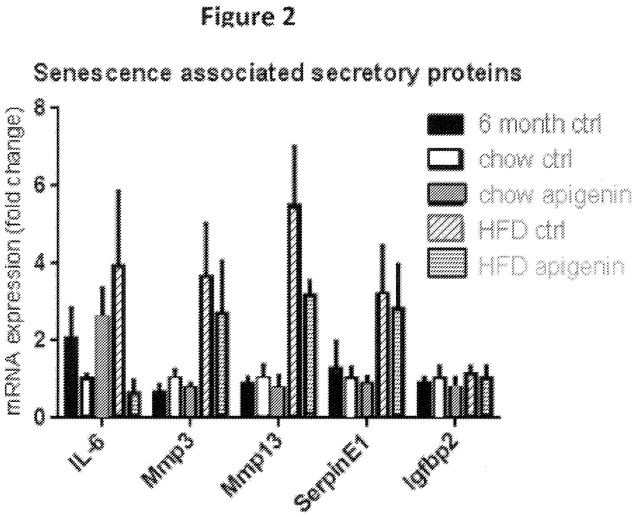

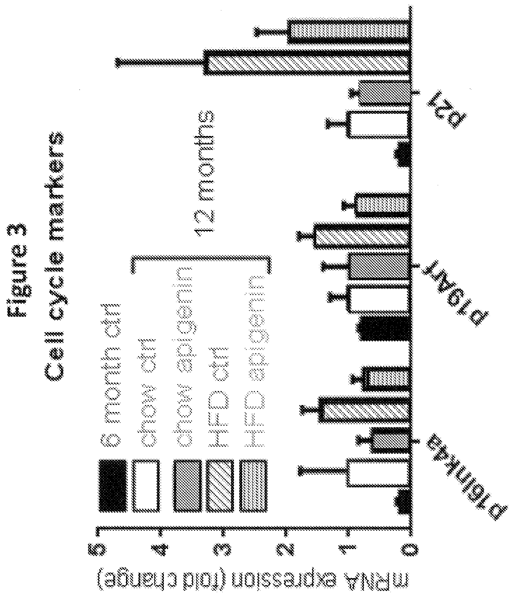

C57BL6 mice were fed chow, chow supplemented with 500 mg/kg apigenin, high fat feed or high fat feed supplemented with 500 mg/kg apigenin for 6 months. At 6 months, mRNA expression levels of senescence associated secretory proteins was determined in epidymal adipose tissue using quantitative RTPCR. The results of the analysis are shown in Figure 2. At 12 months, mRNA expression levels of cell cycle markers was determined in epidymal adipose tissue using quantitative RTPCR. The results of that analysis are shown in Figure 3.

As can be seen from Figures 2 and 3, addition of apigenin to feed resulted in a significant reduction in expression of senescence markers in both chow fed and high fat fed mice.

### Example 3

Expression of p16^{INK4a} functions to limit cell-cycle progression and to promote cellular senescence. Expression of p16^{INK4a} is low in healthy cells, but is induced in cells undergoing senescence. Thus, increases in p16^{lNK4a} expression are associated with cellular senescence and correlate with chronological age in mammals (Burd et al. (2013) Cell 152: 340-351; Krishnamurthy et al. (2004) J. Clin. Invest. 114: 1299).

In order to study the effects of NAD⁺ agonists on cellular senescence associated with the skin, which is associated with inflammation, senescence was induced in a luciferase knockin (p16^{LUC}) mouse (Burd et al. (2013) Cell 152: 340-351) using topical application of Imiquimod. Imiquimod (IMQ) is an immune modulator topically applied for skin conditions such as melanoma and genital warts. The side effect of IMQ is severe, painful inflammation and aging of the skin.

P16^{LUC} mice contain the firefly luciferase cDNA inserted into the translational start site of the endogenous p16^{INK4a} gene. Conditions which induce expression of the p16^{INK4a} gene, and therefore induce cellular senescence, also induce expression of firefly luciferase in p16^{LUC} mice (Burd et al. (2012) Cell 152:340-351; Sorrentino et al. (2014) The Journal of Clinical Investigation, 124(1): 169-351).

The following formulations were prepared:
1. Cream carrier (containing no IMQ) (control or ctrl);
2. IMQ cream (containing 5% IMQ) mixed with 10% water (IMQ); and
3. IMQ cream (containing 5% IMQ) mixed with 500mg/ml NMN (equating to 5% NMN) (IMQ + NMN) .

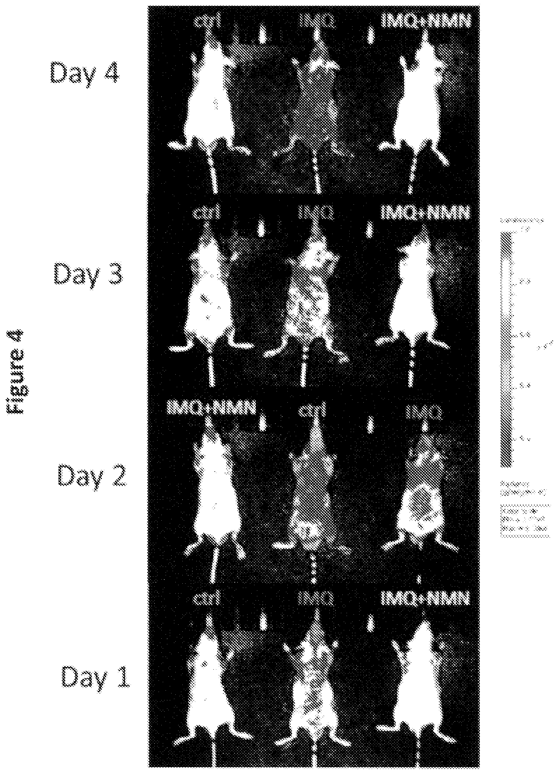

Each of 1, 2 and 3 were applied daily to the rump of SKH1 hairless p16^{LUC} mice for 4 days. The animals were subjected to bioluminescence imaging as a readout for senescence. The results of the imaging are shown in Figure 4. Areas of fluorescence are indicated as darkened areas in Figure 4.

As can be seen from Figure 4, IMQ treated mice showed areas of p16^{lNK4a} induction, and therefore cellular senescence, during the 4 day treatment. In contrast, mice treated with IMQ and the NAD⁺ agonist NMN showed little or no signs of p16^{INK4a} induction, indicating that NMN was able to prevent or reduce cellular senescence.

Mice treated with IMQ and NMN also showed less erythema, scaling and redness than mice treated with IMQ.

These results indicate that topical administration of the NAD⁺ agonist NMN is able to reduce or prevent senescence associated with IMQ treatment and skin aging.

These results indicate that NMN would be useful in topical formulations to treat or prevent cellular senescence associated with skin aging.

### Example 4

Doxorubicin is a chemotherapeutic agent used for the treatment of haematological malignancies, lymphoma, soft tissue sarcomas, and carcinomas. The side effects of doxorubicin include cardiac dysfunction, hair loss, myelosuppression, nausea and vomiting, oral mucositis, oesophagitis, diarrhoea, skin reactions, localised swelling and redness, heart damage and liver dysfunction.

The following formulations were administered to 4 groups of p16^{LUC} mice:
1. Vehicle control: 10% DMSO in PBS administered intraperitoneally;
2. Apigenin: administered in diet at 500mg/kg of food in addition to vehicle control administered i.p.;
3. Doxorubicin: administered at 1 mg/kg body weight intraperitoneally;
4. Doxorubicin + apigenin: Apigenin administered in chow diet at 500mg/kg of food, and doxorubicin administered at 1mg/kg body weight i.p.

Apigenin was administered 4 days prior to administration of doxorubicin.

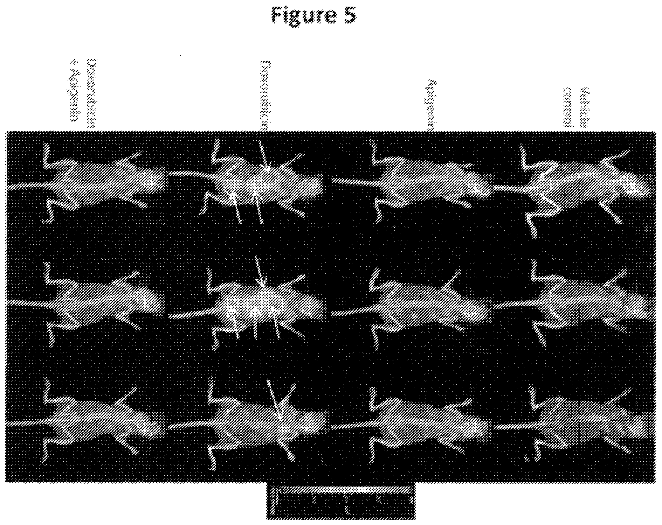

The animals were subjected to bioluminescence/CT imaging (Spectrum-CT instrument, PerkinElmer) as a readout for senescence 24 hours after treatment was completed. The results of the imaging are shown in Figure 5. Areas of luminescence are shown as lightened areas in Figure 5 and are indicated by an arrow.

As can be seen from Figure 5, mice treated with doxorubicin alone showed large areas of bioluminescence indicating induction of p16^{INK4A} and therefore induction of cellular senescence cellular senescence. In contrast, mice treated with a combination of doxorubicin and apigenin showed little or no signs of p16^{INK4A} induction.

These results indicate that apigenin is effective at reducing the induction of senescence by the chemotherapeutic agent doxorubicin.

### Example 5

To study the effects of NAD⁺ agonists on senescence associated with treatment with corticoids, 250g Sprague-Dawley rats were administered daily intraperitoneal injections of the following formulations for 3 days:
1. Vehicle: saline
2. Dexamethasone: 0.8 mg/kg of animal dexamethasone sodium phosphate
3. Dexamethasone + NMN: 0.8 mg/kg body weight dexamethasone sodium phosphate and 1.5 g/kg body weight NMN.

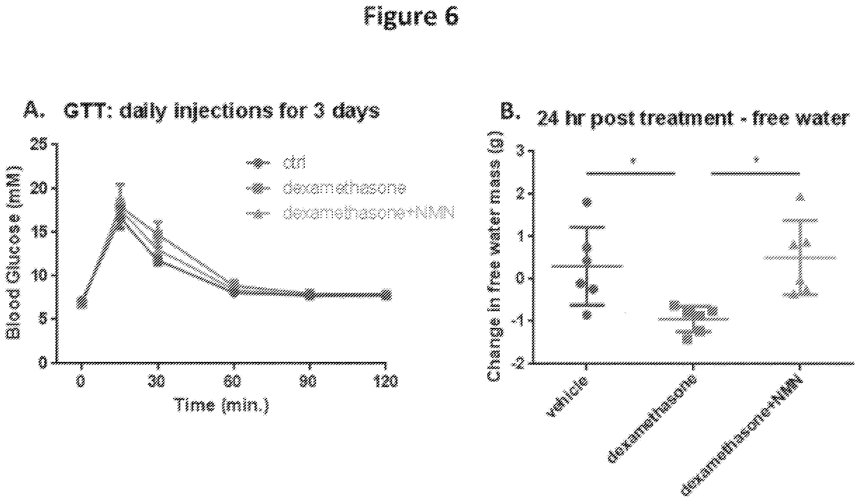

After 3 days, blood glucose was determined. The results of blood glucose analysis are shown in Figure 6A.

Free water was assessed by echMRI one day after injection of the above formulations. The results of free water analysis are shown in Figure 6B.

As can be seen from Figure 6A, dexamethasone had little effect after three days on blood glucose, as did dexamethasone plus NMN. However, administration of dexamethasone significantly reduced the amount of free water, while addition of NMN reversed this effect. Accordingly, NMN was able to reduce water retention caused by dexamethasone.

### Example 6

Senescence associated β-galactosidase (SA-β-Gal) is a biomarker of senescence.

To determine the ability of NAD⁺ agonists to prevent or reduce senescence associated with treatment with corticoids, C2C12 myoblasts were assayed for SA-β-Gal activity following contact with vehicle, NMN, apigenin, dexamethasone, dexamethasone and NMN, and dexamethasone and apigenin. The results are shown in Figures 7A, 7B and 7C. SA-β-Gal activity was determined using the fluorogenic substrate, C12FDG, by flow cytometry (protocol of Debacq-Chainiaux et al. Nature Protocols 2009).
Figure 7A: Cells were treated for 48 hours with either dexamethasone (0.1µg/ml**)**or an ethanol vehicle control, with or without NMN (1mM), n=3. In both the vehicle and dexamethasone treated groups, NMN significantly reduced SA-β-Gal activity.
Figure 7B and 7C: Cells were treated with a pharmaceutical grade dexamethasone sodium phosphate (250nM) with a PBS vehicle control in the presence or absence of NMN (1mM) or apigenin (25uM) for 72 hours (Figure 7B), n = 5-6 and 96 hours (Figure 7C), n = 2. SA-β-Gal activity was decreased with NMN and/or apigenin. All data are presented as the mean value. * p < 0.05, **** p <0.0001, using two-way ANOVA multiple comparisons test.

The results indicate that apigenin and NMN both reduced the percentage of cells which exhibited SA-β-Galactivity following treatment with dexamethasone. These compounds therefore reduce or prevent senescence associated with dexamethasone.

### Example 7

To further investigate the effects of NAD⁺ agonists on dexamethasone induced senescence, 3T3-L1 adipocytes were treated for 5 days with ethanol (control), or 1 mg/ml, 0.5 mg/ml, 0.1 mg/ml or 0.01 mg/ml of dexamethasone in combination with either DMSA, 1mM NMN, 25 mM apigenin, or 1mM NMN and 25mM apigenin and subjected to senescence associated beta-galactosidase staining according to the chromogenic protocol of Debacq-Chainiaux F et al Nature Protocols 2009.

Cells treated with NMN exhibited reduced SA-β-gal staining (blue cells) under all concentrations of dexamethasone.

These results indicate that NMN and apigenin reduce or prevent senescence induced by dexamethasone.

### Example 8

The ability of NAD⁺ agonists to reduce or prevent lipodystrophy associated with anti-retroviral compounds was assessed. The ability of NMN to prevent or reduce lipodystrophy was tested by determining the lipid content of 3T3-L1 adipocytes treated with a vehicle, or the antiretroviral agent stavudine, either alone or in combination with NMN.

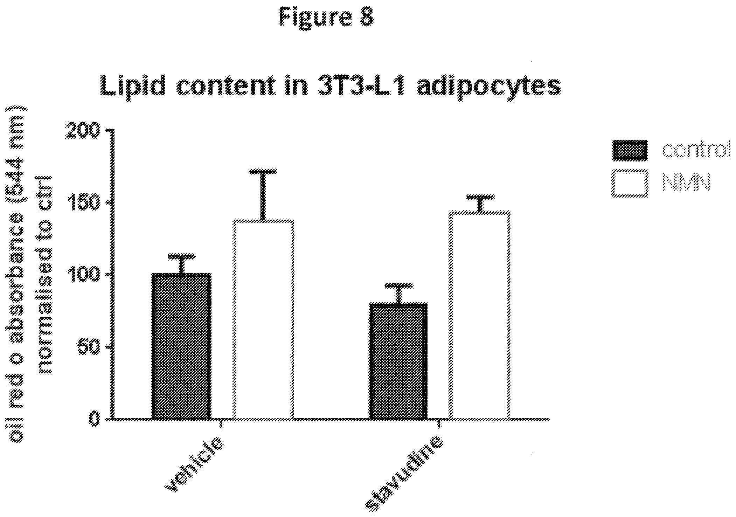

3T3-L1 adipocytes were treated for 24 hr with the the HIV antiretroviral compound stavudine (10 nM) in the presence or absence of NMN (1 mM). Intracellular lipid content was assessed using oil red o staining, which was quantified by reading absorbance at 544 nm. The results are shown in Figure 8. Error bars: SD.

As can be seen from Figure 8, cells treated with stavudine showed a reduction in lipid content compared to control cells treated with vehicle, while cells treated with stavudine and NMN showed no reduction in lipid content compared to cells treated with NMN and vehicle.

These results indicate that NMN can reduce lipodystrophy associated with the anti-retroviral agent stavudine.

### Example 9

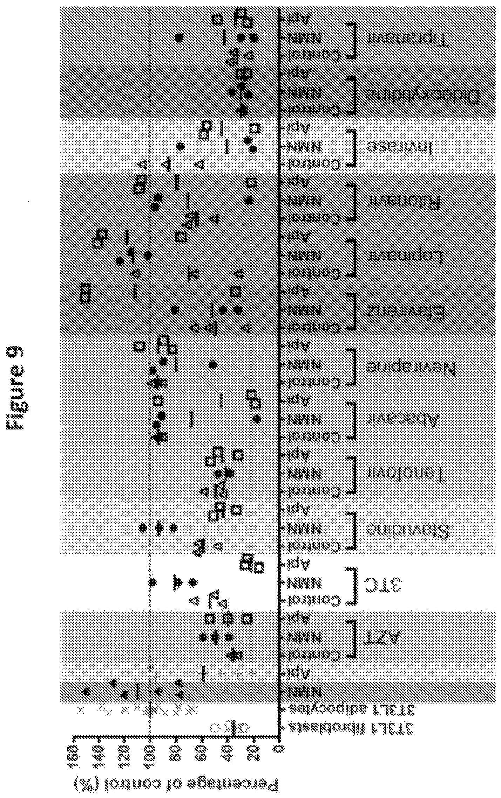

In order to further test the ability of NAD⁺ agonists to reduce lipodystrophy associated with antiretroviral agents, 3T3-L1 adipocytes were treated for 24 hr with various antiretroviral compounds (30 µM) in the presence or absence of NMN (1 mM) or apigenin (25 µM). Intracellular lipid content was assessed using oil red o staining, which was quantified by reading absorbance at 544 nm. The results are shown in Figure 9.

NMN reduced lipodystrophy in cells treated with AZT, 3TC, stavudine, Efavirenz, Lopinavir, Ritonavir, Tipranavir.

Apigenin reduced lipodystrophy in cells treated with AZT, Efavirenz, Lopinavir, and Ritonavir.

### Example 10

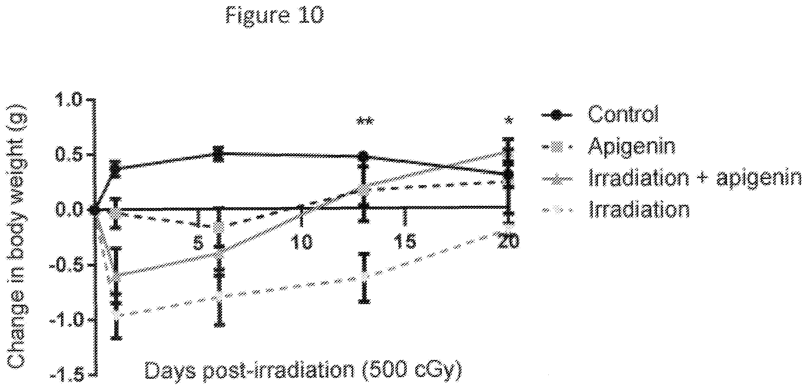

In order to assess the affects of an NAD⁺ agonist on the effects of radiation, female C57BL6 (3-4 months old) were exposed to a single dose (500cGy) of whole body γ-irradiation or no irradiation (control). Mice were then maintained on chow diet with or without the addition of the NAD⁺ raising compound apigenin to diet (500 mg/kg feed). The weight of the mice was determined at various time points for 20 days post-irradiation. The results are shown in Figure 10.

As can be seen from Figure 10, irradiation caused a decrease in body weight, however apigenin prevented this weight loss (*p<0.05, **p < 0.01 irradiated vs irradiated + apigenin, using Kruskal-Wallis multiple comparisons test, n = 3). Results are expressed as mean ± SD.

It is believed that the weight loss is due to a variety of symptoms associated with a decline in health, including for example, nausea, pain, lack of appetite and general malaise.

Accordingly, these results indicate that NAD⁺ agonists can treat or prevent the decline in health, including nausea, pain, lack of appetite and general malaise, associated with irradiation.

### Example 11

The ability of NAD⁺ agonists to reduce senescence associated with various agents was tested by inclubating C2C12 myoblasts with various agents in the presence or absence of NMN or apigenin, and the cells subsequent stained as mentioned above for SA-β-Gal activity.

The following agents were tested:

| **Drug Name** | **Drug Class** |
|---|---|
| Abacavir | Nucleotide analogue reverse transcriptase inhibitors |
| Allopurinol | Xanthine oxidase inhibitor |
| Altretamine | Triazenes |
| Aminolevulinic Acid | Photosensitiser |
| Azacitidine | Hypomethylating agent |
| Azidothymidine/Zidovudine (AZT) | Nucleotide analogue reverse transcriptase inhibitors |
| Bleomycin Sulfate | Streptomycin |
| Bortezomib | Proteasome inhibitor |
| Celecoxib | Asparagine depleters |
| Cispltain | Alkylating agent |
| Cyclophosphamide | Nitrogen mustards |
| Dexamethasone | Corticosteroid |
| Dideoxycytidine | Nucleoside analog reverse-transcriptase inhibitor |
| Doxorubicin HCl | Anthracycline |
| Efavirenz | Non-nucleoside reverse transcriptase inhibitor |
| Etoposide | Topoisomerase II inhibitor |
| Fluorouracil | Antimetabolite |
| Gefitinib | Kinase inhibitor (epidermal growth factor receptor antagonist) |
| Gemcitabine HCl | Ribonucleotide reductase inhibitors |
| Imatinib | Kinase inhibitors |
| Invirase | Protease inhibitor |
| Ixabepilone | Epothilones |
| Lamivudine (3TC) | Nucleotide analogue reverse transcriptase inhibitors |
| Lopinavir | Protease inhibitor |
| Mechlorethamine HCl | Nitrogen mustards |
| Mercaptopurine | Thiopurine |
| Methotrexate | Dihydrofolate reductase inhibitors |
| Mitotane | Not classified |
| Nevirapine | Non-nucleoside reverse transcriptase inhibitor |
| Oxaliplatin | Platinum agent |
| Paclitaxel | Taxane |
| Pentostatin | Adenosine deaminase inhibitors |
| Procarbazine HCl | Hydrazine |
| Quinacrine HCl | Not classified |
| Raloxifene | Estrogen receptor modulator |
| Ritonavir | Protease inhibitor |
| Romidepsin | Histone deacetylase inhibitor |
| Rosiglitazone | Thiazolidinedione |
| Sorafenib | Kinase inhibitors |
| Stavudine | Nucleoside analog reverse-transcriptase inhibitor |
| Tamoxifen Citrate | Estrogen receptor antagonist |
| Tenofovir | Nucleotide analogue reverse transcriptase inhibitors |
| Thiotepa | Aziridine |
| Tipranavir | Nonpeptidic protease inhibitor |
| Topotecan HCl | Topoisomerase inhibitor |
| Tretinoin | Retinoid |
| Vemurafenib | B-RAF inhibitor |
| Vincristine Sulfate | Vinca alkaloid |
| Vismodegib | Hedgehog inhibitor |
| Vorinostat | Histone deacetylase inhibitor |
| Zoledronic Acid | Bisphosphonate |

The proportion of cells which stained blue (i.e. indicating SA-β-Galactivity) was greater in those cells treated with the above agents alone than those cells treated with both the agent and apigenin or the agent and NMN.

These results indicate that both apigenin and NMN were able to reduce the extent of senescence induced by the agents tested.

### Example 12

In order to assess the ability of an NAD⁺ raising compound to reduce the side effects of chemotherapy, body weight was assessed in 4 groups of male C57BL6 mice:
1. Vehicle control: 10% DMSO in PBS administered intraperitoneally;
2. Apigenin: administered in diet at 500 mg/kg of diet, in addition to vehicle control administered i.p.;
3. Doxorubicin: administered at 10 mg/kg body weight intraperitoneally;
4. Doxorubicin + apigenin: Apigenin administered in diet of 500mg/kg of diet, and doxorubicin administered at 10 mg/kg body weight i.p.

Apigenin was administered 8 days prior to administration of a single dose of doxorubicin.

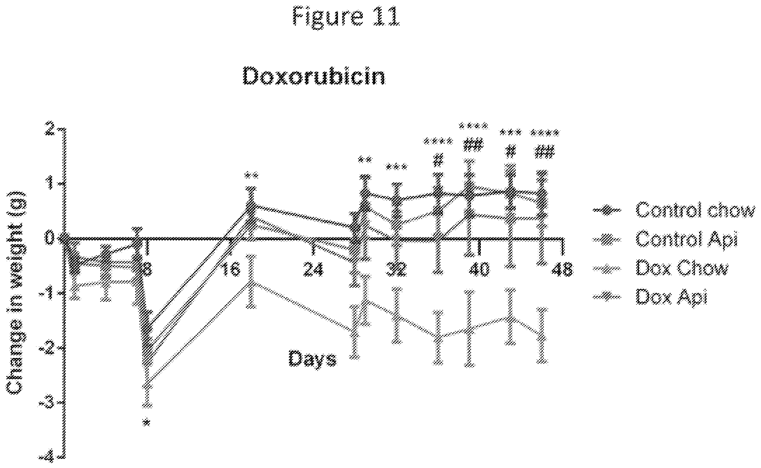

Body weight was assessed during the course of the experiment as a read-out of general health and malaise, the results of which are shown in Figure 11.

As can be seen in Figure 11, intraperitoneal injection of doxorubicin or vehicle control at day 8 causes a reduction in body weight, from which vehicle control injected animals recover. Doxorubicin injection leads to a permanent loss of body weight for at least 40 days, however administration of an NAD⁺ raising compound (apigenin) leads to a rescue in body weight to a degree similar to non-doxorubicin treated animals, showing that NAD⁺ raising compounds can protect against the side effects of chemotherapy, including pain, nausea, lack of appetite and general malaise.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

## Claims

1. An effective amount of a NAD+ precursor selected from NMN (nicotinamide mononucleotide), NaAD (nicotinic acid adenine dinucleotide), NaMN (nicotinic acid mononucleotide), or a pharmaceutically acceptable salt thereof, for use in a method of treating a side effect of chemotherapy and/or radiotherapy in a subject.

2. The NAD+ precursor for use of claim 1, wherein the NAD+ precursor is NMN or a pharmaceutically acceptable salt thereof.

3. The NAD+ precursor for use of claim 1, wherein the NAD+ precursor is NaAD or a pharmaceutically acceptable salt thereof.

4. The NAD+ precursor for use of claim 1, wherein the NAD+ precursor is NaMN or a pharmaceutically acceptable salt thereof.

5. The NAD+ precursor for use of any preceding claim, for use in treating a side effect of radiotherapy in a subject.

6. The NAD+ precursor for use of any preceding claim, for use in treating a side effect of chemotherapy in a subject.

## Patentansprüche

1. Wirksame Menge eines NAD+-Vorläufers, ausgewählt aus NMN (Nikotinamid-Mononukleotid), NaAD (Nikotinsäure-Adenin-Dinukleotid), NaMN (Nikotinsäure-Mononukleotid) oder einem pharmazeutisch akzeptablen Salz davon, zur Verwendung in einem Verfahren zur Behandlung einer Nebenwirkung von Chemo- und/oder Strahlentherapie bei einem Patienten.

2. NAD+-Vorläufer zur Verwendung nach Anspruch 1, wobei der NAD+-Vorläufer NMN oder ein pharmazeutisch akzeptables Salz davon ist.

3. NAD+-Vorläufer zur Verwendung nach Anspruch 1, wobei der NAD+-Vorläufer NaAD oder ein pharmazeutisch akzeptables Salz davon ist.

4. NAD+-Vorläufer zur Verwendung nach Anspruch 1, wobei der NAD+-Vorläufer NaMN oder ein pharmazeutisch akzeptables Salz davon ist.

5. NAD+-Vorläufer zur Verwendung nach einem der vorhergehenden Ansprüche, zur Verwendung bei der Behandlung einer Nebenwirkung von Strahlentherapie bei einem Patienten.

6. NAD+-Vorläufer zur Verwendung nach einem der vorhergehenden Ansprüche, zur Verwendung bei der Behandlung einer Nebenwirkung von Chemotherapie bei einem Patienten.

## Revendications

1. Quantité efficace d'un précurseur de NAD+ choisi parmi NMN (mononucléotide de nicotinamide), NaAD (dinucléotide d'adénine d'acide nicotinique), NaMN (mononucléotide d'acide nicotinique), ou un sel pharmaceutiquement acceptable de ceux-ci, à utiliser dans un procédé de traitement d'un effet secondaire de chimiothérapie et/ou de radiothérapie chez un sujet.

2. Précurseur de NAD+ à utiliser selon la revendication 1, dans lequel le précurseur de NAD+ est NMN ou un sel pharmaceutiquement acceptable de celui-ci.

3. Précurseur de NAD+ à utiliser selon la revendication 1, dans lequel le précurseur de NAD+ est NaAD ou un sel pharmaceutiquement acceptable de celui-ci.

4. Précurseur de NAD+ à utiliser selon la revendication 1, dans lequel le précurseur de NAD+ est NaMN ou un sel pharmaceutiquement acceptable de celui-ci.

5. Précurseur de NAD+ à utiliser selon l'une quelconque des revendications précédentes, destiné à être utilisé dans le traitement d'un effet secondaire de radiothérapie chez un sujet.

6. Précurseur de NAD+ à utiliser selon l'une quelconque des revendications précédentes, destiné à être utilisé dans le traitement d'un effet secondaire de chimiothérapie chez un sujet.
